# EUROPEAN PATENT SPECIFICATION

(11) **EP 2 163 619 B1**
(45) Date of publication and mention of the grant of the patent: **16.05.2012**
(21) Application number: 08777606.8
(22) Date of filing: 19.06.2008
(51) Int. Cl.: C12N 15/09, A01H 5/00

(54) **ROSE CONTAINING FLAVONE AND MALVIDIN, AND METHOD FOR PRODUCTION THEREOF**
ROSE MIT FLAVON UND MALVIDIN UND HERSTELLUNGSVERFAHREN DAFÜR
ROSE CONTENANT UNE FLAVONE ET UNE MALVIDINE, ET SON PROCÉDÉ DE PRODUCTION

(30) Priority: 20.06.2007 JP 2007162959
(43) Date of publication of application: 17.03.2010
(73) Proprietor: Suntory Holdings Limited, Kita-ku, Osaka-shi Osaka 530-8203 (JP)
(72) Inventor: TANAKA, Yoshikazu, Mishima-gun Osaka 618-8503 (JP); KATSUMOTO, Yukihisa, Mishima-gun Osaka 618-8503 (JP); FUKUI, Yuko, Mishima-gun Osaka 618-8503 (JP); MIZUTANI, Masako, Mishima-gun Osaka 618-8503 (JP); NAKAMURA, Noriko, Mishima-gun Osaka 618-8503 (JP); TOGAMI, Junichi, Mishima-gun Osaka 618-8503 (JP)
(74) Representative: Vossius & Partner
(86) International application number: PCT/JP2008/061593
(87) International publication number: WO 2008/156206

(56) References cited:
- EP-A1- 1 067 189
- EP-A1- 1 652 916
- WO-A1-03/062428
- WO-A1-2005/017147
- JP-A- 2000 279 182
- JP-A- 2005 514 950
- TANAKA Y. ET AL.: 'Shokubutsu Shikiso no Seikagaku to Idengaku Hana no Iro no Biotechnology' PROTEIN, NUCLEIC ACID AND ENZYME vol. 47, no. 3, 2002, pages 225 - 230, XP008124957
- FUKUI Y. ET AL.: 'A rationale for the shift in colour towards blue in transgenic carnation flowers expressing the flavonoid 3',5'-hydroxylase gene' PHYTOCHEMISTRY vol. 63, no. 1, 2003, pages 15 - 23, XP004415736
- TANAKA Y. ET AL.: 'Flower colour modification of roses by expression of a torenian anthocyanin methyltransferase gene' PLANT AND CELL PHYSIOLOGY vol. 48, no. SUPPL., August 2007, page S221, ABSTR.NR. 820(P220), XP009134731

## Description

### Technical Field

The invention relates to an artificially made rose containing a flavone and malvidin added by a genetic modification method by expression of an anthocyanin methyltransferase gene, a flavone synthase gene and the pansy *(Viola × wittrockiana)* flavonoid 3',5'-hydroxylase gene. The invention further relates to a method for modifying rose petal color by a co-pigmentation effect which is produced by adding a flovone and malvidin by genetic engineering by expression of an anthocyanin methyltransferase gene, a flavone synthase gene and the pansy *(Viola* × *Wittrockiana)* flavonoid 3',5'-hydroxylasegene, and particularly to a method for altering petal color toward blue.

### Background Art

Flowers are plant reproductive organs that are required for production of seeds for subsequent generations. Formation of seeds requires adhesion of pollen onto the pistils, and fertilization. Pollen is usually carried by insects such as bees and butterflies, by hummingbirds, and rarely by bats. The role of flower petals is to attract these organisms that carry pollen, and plants have developed modifications to flower color, shape and coloring pattern for this purpose.

Since flower color is also the most important trait for ornamental flowers, flowers of various colors have traditionally been bred by cross-breeding. However, it is rare for one plant variety to have different flower colors, and for example, crossbreeding has not produced any purple to blue varieties for rose (*Rosa hybrida*), carnation (*Dianthus caryophyllus*), chrysanthemum (*Chrysanthemum morifolium*) or lily (*Lilium* spp.), or bright red varieties for Japanese garden iris (*Iris ensata* Thumb.) or gentian (*Gentiana triflora*).

Light yellow to red or blue flower colors are generally due to the presence of flavonoids and anthocyanins (colored glucosides belonging to the flavonoid class). Flavonoids are common secondary metabolites of plants, and they have a basic C₆C₃C₆ backbone and are synthesized from phenylalanine and malonyl-CoA, as shown below. They are classified as flavones, flavonols, etc. according to the oxidation states of the C-rings.

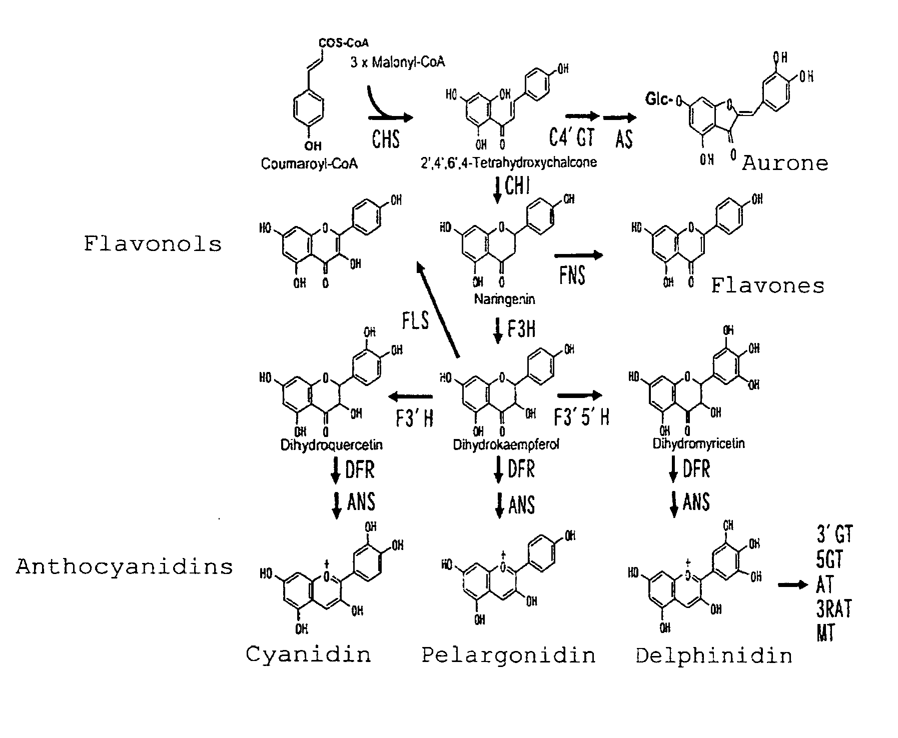

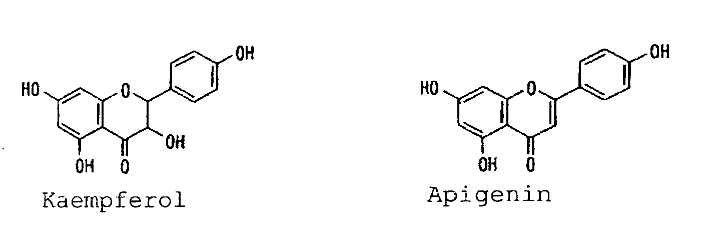

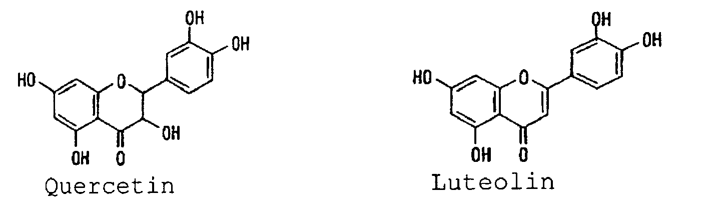

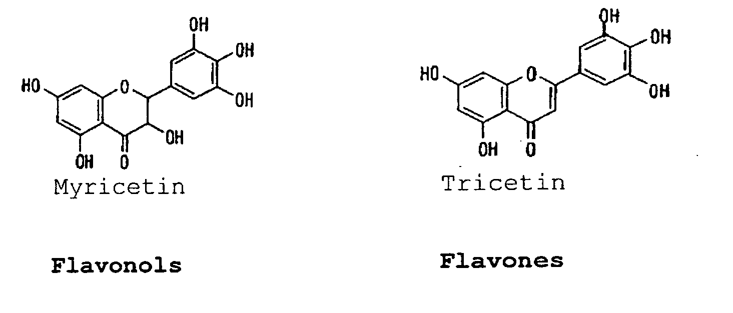

Flavonoids absorb ultraviolet rays and remove radicals, and their original function is therefore believed to be protection of plant bodies from various forms of stress. They have also received attention in recent years as healthy components (see Harborne and Williams 2000 Phytochemistry 55, 481-504).

Several hundred molecular species of colored anthocyanins are known, and of the chromophoric anthocyanidins, the most common are the following 6 types: (1) pelargonidin abundant in orange to red flowers, (2) cyanidin and peonidin abundant in red to crimson flowers, and (3) delphinidin, petunidin and malvidin abundant in violet to blue flowers.

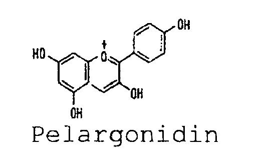

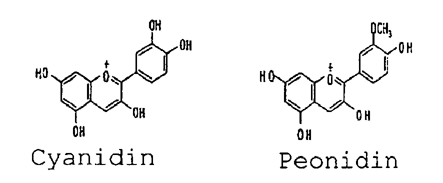

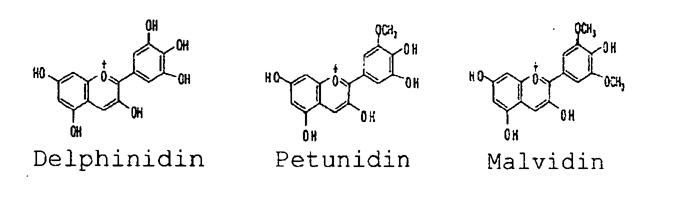

The anthocyanin structure has a major effect on color. An increased number of hydroxyl groups on the B ring of the anthocyanin results in a greater degree of blue. Delphinidin-type anthocyanins are bluer than pelargonidin-type anthocyanins and cyanidin-type anthocyanins. Biosynthesis of flavonoids including anthocyanins is highly conserved across plant species. Flavonoids are biosynthesized in the cytosol, and after addition of sugars and acyl groups, they are transported to the vacuoles and accumulated (see Tanaka et al. 2005 Plant Cell, Tissue and Organ Culture 80,1-24 and Tanaka and Brugliera 2006 Ainsworth, ed. Flowering and its manipulation, pp.201-239, Blackwell Publishing Ltd.).

The structural genes of the enzymes involved in the biosynthesis have all been cloned. Creating recombinant plants therefore allows modification of the structures and amounts of flavonoids that are accumulated in flowers by artificial expression of their genes, thereby altering the flower color (Tanaka et al. 2005 Plant Cell, Tissue and Organ Culture 80,1-24, Tanaka and Brugliera 2006 Ainsworth, Flowering and its manipulation, pp.201-239, Blackwell Publishing Ltd.). For example, for carnations or roses that cannot produce delphinidin in the petals, the flavonoid 3',5'-hydroxylase (hereinafter abbreviated as "F3'5'H") gene necessary for synthesis of delphinidin has been expressed to produce delphinidin to create an artificial blue flower (see Tanaka 2006 Phytochemistry Reviews 5, 283-291).

Such methods of artificially modifying plant metabolism are sometimes called "metabolic engineering". In order to modify metabolism for accumulation of a substance of interest expression of the gene of the enzyme that produces the substance of interest in a recombinant plant is possible, but in many cases competition with endogenous enzymes of the same plant results in little or absolutely no accumulation of the substance of interest, and therefore no industrially useful trait is obtained.

For example, petunias (*Petunia hybrida*) do not accumulate pelargonidin due to the specificity of dihydroflavonol reductase (hereinafter abbreviated as "DFR"), and therefore no natural varieties exist with orange-colored flowers.

While orange petunias that accumulate pelargonidin by transfer of the DFR gene from roses or the like have been reported, the accumulation of pelargonidin requires the use of petunia varieties lacking the genes for flavonoid 3'-hydroxylase (hereinafter abbreviated as "F3'H"), F3'5'H and flavonol synthase (hereinafter abbreviated as "FLS") that compete with DFR, because no change in phenotype is observed when the rose DFR gene is transferred into petunias that do not lack these genes (see Tanaka and Brugliera 2006 Ainsworth, Flowering and its manipulation, pp.201-239, Blackwell Publishing Ltd.). Consequently, it cannot be predicted whether a compound of interest will be accumulated to exhibit the desired phenotype simply by transferring a gene of interest.

In addition, metabolic engineering often produces unpredictable results. For example, when expression of the flavone synthase gene was inhibited in torenia (*Torenia hybrida*), the flavone content was reduced and accumulation of flavanones was observed. Accumulation of flavanones would be expected to result in an increased anthocyanin content, but in actuality the anthocyanin content decreased (Ueyama et al. Plant Science, 163, 253-263, 2002). It is therefore difficult to predict changes in metabolites, and persistent modifications have been necessary to obtain desired phenotypes.

Anthocyanins bound with higher numbers of aromatic acyl groups also appear bluer due to an intramolecular copigment effect. Anthocyanins with two or more aromatic acyl groups are known as polyacylated anthocyanins, and they exhibit a stable blue color (see Harborne and Williams 2000 Phytochemistry 55, 481-504).

The color of a flower changes not only by the structure of the anthocyanin pigments themselves as the essential pigments, but also due to copresent flavonoids (also known as copigments), metal ions, and the pH of the vacuoles. For example, flavones or flavonols are typical copigments that form sandwich-like stacking with anthocyanins and render the anthocyanins bluing and deepening color effects (see Goto (1987) Prog. Chem. Org. Natl. Prod. 52). Flavones can thus be considered colorless copigment components. For example, isovitexin, a type of flavone, exhibits a copigment effect for anthocyanins in Japanese garden iris (*Iris ensata* Thunb.). Isovitexin also stabilizes anthocyanins, thus producing a stabilizing effect on Japanese garden iris flower color (see Yabuya et al. Euphytica 2000 115, 1-5).

Flavones usually exhibit stronger copigment effects than flavonols. For example, analysis of genetically modified carnations has indicated a stronger copigment effect for flavones than flavonols (see Fukui et al. Phytochemistry, 63, 15-23, 2003). Accumulation of flavones is therefore important for creating blue flower color. However, not all plants can produce flavones, and it is known that roses and petunias do not accumulate flavones. In addition to flower color, it is known that flavones play a role in absorption of ultraviolet rays, countering various types of stress, and interaction with microorganisms, and that plants with new traits can be obtained through synthesis of flavones (as a patent document relating to a gene coding for flavone synthase, see Japanese Unexamined Patent Publication No. 2000-279182). However, as yet no examples of flavone-expressing roses have been known.

Flavones are synthesized from flavanones by reaction catalyzed by flavone synthase. Specifically, apigenin is synthesized from naringenin, luteolin is synthesized from eriodictyol and tricetin is synthesized from pentahydroxyflavanone. Flavone synthase exists in two forms, flavone synthase I and flavone synthase II. Both catalyze the same reaction, but are different types of enzymes. Flavone synthase I is a 2-oxoglutaric acid-dependent dioxygenase (see Britsch et al. (1981) Z. Naturforsch 36c pp. 742-750 and Britsch (1990) Arch. Biochem. Biophys. 282 pp. 152-160), while flavone synthase II is a cytochrome P450-type monooxygenase. The structural gene of flavone synthase II can be obtained from torenia, snapdragon, perilla (*Perilla frutescens*), gerbera (*Gerbera hybrida*) and gentian (see Tanaka and Brugliera 2006 Ainsworth, Flowering and its manipulation, pp.201-239, Blackwell Publishing Ltd.).

Flavone synthesis is predictable when the flavone synthase gene is expressed in genetically modified plants that do not produce flavones. However, when the torenia flavone synthase gene is expressed in petunias, it has been reported that the deep violet color of the flower becomes faint (Tsuda et al. Plant Biotechnology, 21, 377-386, 2004). It has also been reported that expression of the gentian-derived flavone synthase gene in tobacco results in flavone synthesis but, likewise, results in a fainter flower color (Nakatsuka et al. 2006, Molecular Breeding 17:91-99). Thus, blue flower color is not always obtained even when flavones are synthesized. The reason for the lack of copigment effect could be an unsuitable ratio of the anthocyanin and flavone contents or unsuitable modification of the anthocyanins and flavones with sugars and acyl groups. These results suggest that it is not possible to increase the blueness of flower color simply by expressing the flavone synthase gene and accumulating flavones.

Roses are the most popular of flowering plants, and they have been cultivated since ancient times. Artificially modified varieties have also been produced in the past several hundred years. Roses have therefore been obtained containing flavonoids such as pelargonidin, cyanidin and flavonols. In recent years as well, roses have been created by genetic modification techniques to produce delphinidin that is not naturally found in roses. However, no flavone-accumulating roses have yet been obtained, either by cross-breeding or by genetic modification. In addition, no roses have yet been obtained that accumulate both a flavone and malvidin.

### Disclosure of the Invention

The major advantage of using genetic modification for breeding of plants is that, unlike cross-breeding, it allows modifications to plants that cannot be achieved by cross-breeding, and modifications using genes from other organisms. That is, genetic modification allows any gene of an organism of a different species to be transferred into a plant such as a rose, to impart a new ability to the plant. However, unlike model plants such as Arabidopsis (*Arabidopsis thaliana*) and tobacco (*Nicotiana tabacum* L.), the functioning of transferred genes in roses is largely dependent on the source of the gene and the promoter used.

According to WO2005/017147 transfer of the flavonoid 3',5'-hydroxylase gene (F3'5'H) into roses resulted in no expression in the genetically modified rose and no detection of delphinidin when the gene was derived from petunia or gentian, but interestingly, when the gene was derived from pansy it was expressed and imparted to roses the new ability to produce delphinidin. In roses, therefore, it cannot be easily inferred which genes derived from which plant varieties will function when transferred.

When a gene is transferred into chrysanthemums as well, it is difficult to predict whether the gene will function in the chrysanthemums, and it is known that transferred genes lose their function as recombinant chrysanthemums age. The 35S promoter of cauliflower mosaic virus, which is often used for transfer of foreign genes in recombinant plants, has been reported to function in gentian (see Mishiba et al. Plant Journal 2005, 44:541-556).

While it can be assumed that synthesis of flavones in roses can be easily achieved by expressing the flavone synthase gene, it is not easy to predict whether to express the dioxygenase-type or the cytochrome P450-type flavone synthase, or which plant source should be used for the flavone synthase gene, and therefore trial and error is necessary. The copigment effect is a phenomenon produced when anthocyanins and flavones or flavonols are copresent in a certain concentration in the vacuoles, and it has been demonstrated that this requires the flavone or flavonol copigments to undergo glycosylation or other modification more adapted to the condition of glycosylation or other modification of the anthocyanin color sources (see Nature. 2005 Aug 11; 436(7052):791 and Nature, 358, 515-518 (1992)).

For expression of the necessary color tone it is necessary for the anthocyanins and flavones/flavonols to be in the optimal structural combination, and this requires trial and error in regard to what sort of modifications should exist in the copresent anthocyanins and flavones/flavonols. In addition, because flavanones such as naringenin are rapidly hydroxylated by flavanone 3-hydroxylase (hereinafter abbreviated as "F3H") in natural roses, flavones are not necessarily synthesized from flavanones even if flavone synthase is functioning in the rose.

It is therefore an object of the invention to provide roses comprising appropriate pigments for expression of desired color tones in the roses.

As a result of much research directed toward solving the problems mentioned above, the inventors have completed this invention upon finding that desired color tone expression can be accomplished by artificially adding flavones and malvidin to roses.

Specifically, the present invention provides the following:
1. A rose characterized by comprising a flavone and malvidin added by a genetic modification method
   by expression of an anthocyanin methyltransferase gene, a flavone synthase gene and the pansy *(Viola* × *wittrockiana*) flavonoid 3',5'-hydroxylase gene.
2. A rose according to 1,
   wherein the flavone synthase gene is a flavone synthase gene derived from the family Scrophulariaceae.
3. A rose according to 2, wherein the flavone synthase gene derived from the family Scrophulariaceae is a flavone synthase gene derived from snapdragon of the family Scrophulariaceae (Scrophulariaceae, *Antirrhinum majus*).
4. A rose according to 2 above, wherein the flavone synthase gene derived from the family Scrophulariaceae is a flavone synthase gene derived from torenia of the family Scrophulariaceae (Scrophulariaceae, *Torenia hybrida*).
5. A rose according to 3, wherein the flavone synthase gene derived from snapdragon of the family Scrophulariaceae is a gene coding for:
   (1) flavone synthase having the amino acid sequence listed as SEQ ID NO: 2,
   (2) flavone synthase having the amino acid sequence listed as SEQ ID NO: 2 modified by an addition or deletion of one or several amino acids and/or substitution of one or several amino acids by other amino acids,
   (3) flavone synthase having an amino acid sequence with at least 90% sequence identity to the amino acid sequence listed as SEQ ID NO: 2, or
   (4) flavone synthase encoded by nucleic acid that hybridizes with nucleic acid having the nucleotide sequence of SEQ ID NO: 1 under highly stringent conditions.
6. A rose according to 4, wherein the flavone synthase gene derived from torenia of the family Scrophulariaceae is a gene coding for:
   (1) flavone synthase having the amino acid sequence listed as SEQ ID NO: 4,
   (2) flavone synthase having the amino acid sequence listed as SEQ ID NO: 4 modified by an addition or deletion of one or several amino acids and/or substitution of one or several amino acids by other amino acids,
   (3) flavone synthase having an amino acid sequence with at least 90% sequence identity to the amino acid sequence listed as SEQ ID NO: 4, or
   (4) flavone synthase encoded by nucleic acid that hybridizes with nucleic acid having the nucleotide sequence of SEQ ID NO: 3 under highly stringent conditions.
7. A rose according to any one of 1 to 6,
   wherein the pansy flavonoid 3',5'-hydroxylase gene is a gene coding for:
   (1) flavonoid 3',5'-hydroxylase having the amino acid sequence listed as SEQ ID NO: 8,
   (2) flavonoid 3',5'-hydroxylase having the amino acid sequence listed as SEQ ID NO: 8 modified by an addition or deletion of one or several amino acids and/or substitution of one or several amino acids by other amino acids,
   (3) flavonoid 3',5'-hydroxylase having an amino acid sequence with at least 90% sequence identity to the amino acid sequence listed as SEQ ID NO: 8, or
   (4) flavonoid 3',5'-hydroxylase encoded by nucleic acid that hybridizes with nucleic acid having the nucleotide sequence of SEQ ID NO: 7 under highly stringent conditions.
8. A rose according to any one of 1 to 7, wherein the anthocyanin methyltransferase gene is a gene coding for:
   (1) methyltransferase having the amino acid sequence listed as SEQ ID NO: 10,
   (2) methyltransferase having the amino acid sequence listed as SEQ ID NO: 10 modified by an addition or deletion of one or several amino acids and/or substitution of one or several amino acids by other amino acids,
   (3) methyltransferase having an amino acid sequence with at least 90% sequence identity to the amino acid sequence listed as SEQ ID NO: 10, or
   (4) methyltransferase encoded by nucleic acid that hybridizes with nucleic acid having the nucleotide sequence of SEQ ID NO: 9 under highly stringent conditions.
9. A rose according to any one of 1 to 8 wherein the flower color is changed with respect to the host
10. A rose according to 9, wherein the change in flower color is a change toward blue.
11. A rose according to 9 or 10, wherein the change in flower color is a change such that the hue angle (θ) according to the L*a*b color system chromaticity diagram approaches 270° which is the blue axis.
12. A rose according to 9, wherein the change in flower color is a change such that the minimum value of the reflection spectrum of the petal shifts toward the longer wavelength end.
13. A rose portion, descendant, tissue, vegetative body or cell having the same properties as a rose according to any one of 1 to 12.
14. A method for modifying the flower color of a rose by a co-pigmentation effect produced by adding a flavone and malvidin by a gnetic modification technique by expression of an anthocyanic methyltransferase gene, a flavone synthase gene and the pansy *(Viola x wittrockiana)* flavonoid 3',5'-hydroxylase gene.
15. The method according to 14, wherein the co-pgimentation effect is an effect of changing the flower color toward blue.

### Best Mode for Carrying Out the Invention

### Definition of terms

The term "rose", as used throughout the present specification, is a general name for an ornamental plant which is a deciduous shrub of the order Rosales, family Rosaceae, genus *Rosa*, and it is not limited to any specific variety and includes the entire plant or a portion thereof usually containing the flower.

A reference to a "portion, descendant, tissue, vegetative body or cell" of a "rose", as used throughout the present specification, means any thing derived from a "rose" so long as it retains the desired genetic trait of a "rose" according to the invention, and it is not limited to any particular entity.

The phrase "highly stringent conditions", as used throughout the present specification means, for example, conditions of heating the antisense strand and the target nucleic acid overnight at 55°C in a solution comprising 6 × SSC (1 × SSC composition: 0.15 M NaCl, 0.015 M sodium citrate, pH 7.0), 0.5% SDS, 5 × Denhardt, 100 µg/ml denatured fragmented salmon sperm DNA and 50% formamide, and rinsing under conditions of 0.1 × SSC and/or conditions of 60°C or above, and specifically it refers to any conditions under which the nonspecific signal of the background is essentially absent.

The phrase "hue angle (θ) according to the L*a*b color system chromaticity diagram", as used throughout the present specification, refers to the hue angle (θ) standardized by the 1976 Commission internationale de l'eclairage (CIE) and adopted in Japan as JIS8729, where 0° is the red direction, 90° is the yellow direction, 180° is the green direction and 270° is the blue direction. Flower color can be represented by a combination of this hue angle and RHS (Royal Horticultural Society) color chart data.

### Transfer of flavone synthesis gene, flavonoid 3',5'-hydroxylase gene and anthocyanin methyltransferase gene

The gene for flavone synthase II derived from perilla was transferred into rose by a known procedure, together with the pansy F3'5'H gene. As a result, no flavones were detected in roses into which the perilla flavone synthase II gene had been transferred, indicating that the gene does not function in rose. On the other hand, flavone was detected in roses into which torenia or snapdragon flavone synthase II genes had been transferred, indicating that the flavone synthesis genes do function in rose.

Flavone-accumulating roses not found in the prior art were thus created. The flavone content (%) of the total flavonoids may be 1% or greater, preferably 5% or greater, more preferably 10% or greater and most preferably 30% or greater. Roses accumulating both anthocyanins and flavones have relatively bluer colors compared to roses containing only the same anthocyanins, thus suggesting that flavone accumulation contributes to the new trait of blueness.

In addition, it was found that when an anthocyanin with a methylated B ring (anthocyanins including malvidin) and a flavone are copresent, a higher copigment effect is exhibited than when a delphinidin-containing anthocyanin and a flavone are copresent, and that by transferring a methyltransferase gene for the B ring in addition to the flavone synthase II gene and pansy F3'5'H gene, it is possible to accumulate methylated delphinidin-type anthocyanins and flavones in rose petals, thus resulting in a bluer color of the rose petals.

By a hybridization test it was also found that the trait of accumulating both delphinidin-type anthocyanins and flavones is transmitted to progeny.

These findings indicate that in plants that do not accumulate flavones or do not accumulate methylated anthocyanins such as malvidins in the petals, a bluer color shade of the petals can be produced by causing these compounds to be accumulated simultaneously. For this purpose it is preferable for the host to be a plant that normally does not accumulate flavones or malvidin, such as a rose.

The enzymes described herein are typically enzymes having specific amino acid sequences listed in the Sequence Listing. However, it is well known that desired enzyme activity can be maintained not only with the natural amino acid sequence of an enzyme, but also with the same amino acid sequence having modifications in regions other than the regions associated with the enzyme activity. Consequently, the enzymes described herein include proteins having the amino acid sequences specified by the SEQ ID NOs which are modified by an addition or deletion of one or several amino acids and/or by substitution of one or several amino acids with other amino acids, and still maintaining the original enzyme activities, and also proteins having amino acid sequences with at least 90% sequence identity to the specific amino acid sequences specified by the SEQ ID NOs, and maintaining the original enzyme activities.

It is known that for any gene coding for a certain enzyme, there is a high probability that nucleic acid that hybridizes with the gene under highly stringent conditions will code for an enzyme having the same activity as that enzyme. Thus, enzymes encoded by nucleic acids that hybridize with nucleic acids having the nucleotide sequences specified by the SEQ ID NOs under highly stringent conditions, and having the desired enzyme activities, are also included as enzymes described herein.

The following genes may therefore be mentioned as enzyme genes described herein.

### (A) Snapdragon (Antirrhinum majus) flavone synthase gene

A gene coding for:
(1) flavone synthase having the amino acid sequence listed as SEQ ID NO: 2,
(2) flavone synthase having the amino acid sequence listed as SEQ ID NO: 2 modified by an addition or deletion of one or several amino acids and/or substitution of one or several amino acids by other amino acids,
(3) flavone synthase having an amino acid sequence with at least 90% sequence identity to the amino acid sequence listed as SEQ ID NO: 2, or
(4) flavone synthase encoded by nucleic acid that hybridizes with nucleic acid having the nucleotide sequence of SEQ ID NO: 1 under highly stringent conditions.

### (B) Torenia (Torenia hybrida) flavone synthase gene

A gene coding for:
(1) flavone synthase having the amino acid sequence listed as SEQ ID NO: 4,
(2) flavone synthase having the amino acid sequence listed as SEQ ID NO: 4 modified by an addition or deletion of one or several amino acids and/or substitution of one or several amino acids by other amino acids,
(3) flavone synthase having an amino acid sequence with at least 90% sequence identity to the amino acid sequence listed as SEQ ID NO: 4, or
(4) flavone synthase encoded by nucleic acid that hybridizes with nucleic acid having the nucleotide sequence of SEQ ID NO: 3 under highly stringent conditions.

### (C) Perilla (Perilla frutescens) flavone synthase gene

A gene coding for:
(1) flavone synthase having the amino acid sequence listed as SEQ ID NO: 6,
(2) flavone synthase having the amino acid sequence listed as SEQ ID NO: 6 modified by an addition or deletion of one or several amino acids and/or substitution of one or several amino acids by other amino acids,
(3) flavone synthase having an amino acid sequence with at least 90% sequence identity to the amino acid sequence listed as SEQ ID NO: 6, or
(4) flavone synthase encoded by nucleic acid that hybridizes with nucleic acid having the nucleotide sequence of SEQ ID NO: 5 under highly stringent conditions.

### (D) Pansy (Viola x wittrockiana) 3',5'-hydroxylase gene

A gene coding for:
(1) 3',5'-hydroxylase having the amino acid sequence listed as SEQ ID NO: 8,
(2) 3',5'-hydroxylase having the amino acid sequence listed as SEQ ID NO: 8 modified by an addition or deletion of one or several amino acids and/or substitution of one or several amino acids by other amino acids,
(3) 3',5'-hydroxylase having an amino acid sequence with at least 90% sequence identity to the amino acid sequence listed as SEQ ID NO: 8, or
(4) 3',5'-hydroxylase encoded by nucleic acid that hybridizes with nucleic acid having the nucleotide sequence of SEQ ID NO: 7 under highly stringent conditions.

### (E) Torenia (Torenia hybrida) methyltransferase gene

A gene coding for:
(1) methyltransferase having the amino acid sequence listed as SEQ ID NO: 10,
(2) methyltransferase having the amino acid sequence listed as SEQ ID NO: 10 modified by an addition or deletion of one or several amino acids and/or substitution of one or several amino acids by other amino acids,
(3) methyltransferase having an amino acid sequence with at least 90% sequence identity to the amino acid sequence listed as SEQ ID NO: 10, or
(4) methyltransferase encoded by nucleic acid that hybridizes with nucleic acid having the nucleotide sequence of SEQ ID NO: 9 under highly stringent conditions.

### Examples

The invention will now be explained in greater detail by the following examples.

### Example 1: Simulation of flavone copigment effect with anthocyanins

Anthocyanins were prepared first for simulation of the flavone copigment effect with anthocyanins. Cyanin was extracted and purified from petals of the rose variety "Rote Rose" (rose cv. "Rote Rose"). Delphin was obtained by alkali hydrolysis of the pigment extracted from petals of the verbena variety "Tapien Violet" (verbena cv. "Tapien Violet" or verbena variety Sunmaref TP-V ("Tapien Violet")("Tapien", is a Trade Mark registered in Japan)), followed by purification. Malvin and luteolin 7-O-glucoside were purchased from Funakoshi Corp.

The flavone (luteolin 7-O-glucoside) was added to each anthocyanin prepared in this manner, at 0, 1, 2 and 4 equivalent molar concentrations in a buffering solution at pH 5.0, and the absorption spectra were measured. The anthocyanins used were cyanin (cyanidin 3,5-diglucoside), delphin (delphinidin 3,5-diglucoside) and malvin (malvidin 3,5-diglucoside). The anthocyanin concentrations for cyanin, delphin and malvin were 1 mM.

As shown in Tables 1 and 2, addition of the flavone increased the absorbance of the anthocyanin aqueous solutions and the degree of change (absorbance ratio) was greatest with malvin. The absorption maxima (λmax) were also shifted toward the long wavelength end with addition of the flavone. The degree of change was greatest with malvin, and then with delphin. Upon evaluation of the color shade value based on the L*a*b* color system, addition of the flavone was found to produce a bluer color shade and increased chroma. This effect was most notable with malvin. That is, it was demonstrated that the luteolin 7-O-glucoside copigment effect was exhibited to the greatest extent with malvin.

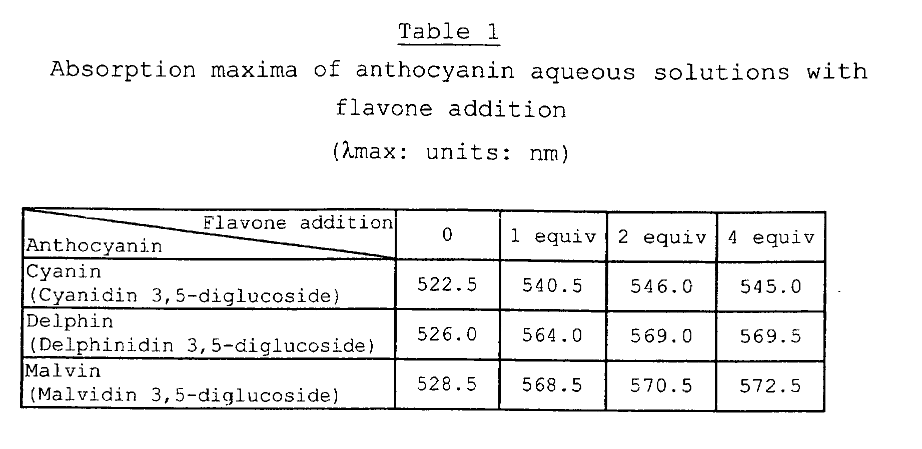

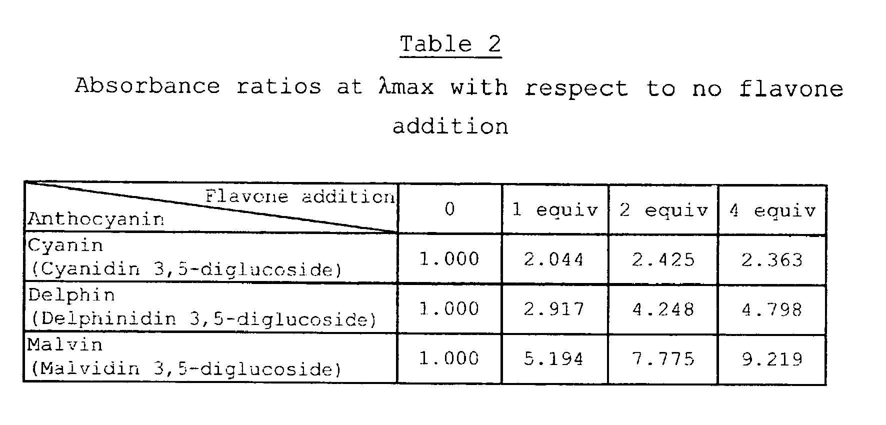

### Example 2 (reference example): Transfer of pansy F3'5'H#40 gene and perilla flavone synthase gene into rose variety "Lavande"

The perilla flavone synthase gene-containing plasmid pYFS3 described in Japanese Unexamined Patent Publication No. 2000-279182 was digested with XbaI and then blunt ended and digested with BamHI to obtain an approximately 1.8 kb perilla flavone synthase gene fragment. Separately, pSPB906 described in WO2005/01714 was digested with XhoI and then blunt ended and further digested with BamHI. The perilla flavone synthase gene fragment was inserted between the flush ends and the BamHI cleavage site to obtain plasmid 906-pYFS3. Plasmid 906-pYES3 comprises the perilla flavone synthase gene between the El₂35S promoter and D8 terminator (both described in WO2005/017147).

A plasmid obtained by inserting a fragment of the pansy F'3"5"H#40 gene, cut out from pCGP1961 described in WO2005/017147 by partial digestion with BamHI and XhoI, at the BamHI and SalI sites of pSPB176 reported by Ueyama et al. (Ueyama et al. Plant Science, 163, 253-263, 2002), was designated as pSPB575. At the AscI site of this plasmid there was inserted an approximately 3.4 kb perilla flavone synthase gene expression cassette obtained by digesting the aforementioned plasmid 906-pYFS3 with AscI. Of the obtained plasmids, the vector having the F3'5'H#40 gene expression cassette and the perilla flavone synthase expression cassette linked in the same direction was designated as pSPB1310. This plasmid constitutively expresses the pansy F3'5'H#40 gene and the perilla flavone synthase gene in plants.

Plasmid pSPB1310 constructed in this manner was transferred into the mauve rose variety "Lavande", and 55 transformants were obtained. Delphinidin accumulation was confirmed in 49 of 50 pigment-analyzed transformants, with a maximum delphinidin content of 70% (average: 26%). However, absolutely no flavones were detected, and it was therefore concluded that the perilla flavone synthase gene does not function in rose cells.

The analysis values for representative transformants are shown in Table 3 below.

**Table 3**

| Plant No. | Del (%) | Anthocyanidin (mg/g) | | | Flavonol (mg/g) | | | Flavone (mg/g) | | | |
|---|---|---|---|---|---|---|---|---|---|---|---|
| | | Del | Cya | Pel | M | Q | K | Tri | Lut | Api | Total |
| Control | 0.0 | 0.000 | 0.078 | 0.000 | 0.000 | 0.451 | 0.078 | 0.000 | 0.000 | 0.000 | 0.000 |
| 1 | 70.3 | 0.105 | 0.045 | 0.000 | 0.253 | 0.152 | 0.017 | 0.000 | 0.000 | 0.000 | 0.000 |
| 2 | 67.1 | 0.098 | 0.048 | 0.000 | 0.379 | 0.291 | 0.026 | 0.000 | 0.000 | 0.000 | 0.000 |
| 3 | 50.7 | 0.060 | 0.058 | 0.000 | 0.326 | 0.289 | 0.013 | 0.000 | 0.000 | 0.000 | 0.000 |
| 4 | 60.6 | 0.050 | 0.033 | 0.000 | 0.216 | 0.188 | 0.007 | 0.000 | 0.000 | 0.000 | 0.000 |
| 5 | 66.1 | 0.073 | 0.037 | 0.000 | 0.608 | 0.380 | 0.045 | 0.000 | 0.000 | 0.000 | 0.000 |
| 6 | 67.7 | 0.055 | 0.026 | 0.000 | 0.536 | 0.319 | 0.039 | 0.000 | 0.000 | 0.000 | 0.000 |
| 7 | 56.9 | 0.062 | 0.047 | 0.000 | 0.253 | 0.201 | 0.009 | 0.000 | 0.000 | 0.000 | 0.000 |
| 8 | 52.5 | 0.109 | 0.099 | 0.000 | 0.307 | 0.438 | 0.034 | 0.000 | 0.000 | 0.000 | 0.000 |
| 9 | 50.4 | 0.073 | 0.072 | 0.000 | 0.281 | 0.362 | 0.013 | 0.000 | 0.000 | 0.000 | 0.000 |
| 10 | 61.9 | 0.085 | 0.052 | 0.000 | 0.228 | 0.192 | 0.008 | 0.000 | 0.000 | 0.000 | 0.000 |

| | | | | | | | | | | | |
|---|---|---|---|---|---|---|---|---|---|---|---|
| Control: Lavande control Del: Delphinidin, Cya: Cyanidin, Pel: Pelargonidin, M: Myricetin, Q: Quercetin, K: Kaempferol, Tri: Tricetin, Lut: Luteolin, Api: Apigenin Del(%): Proportion of delphinidin in total anthocyanidins | | | | | | | | | | | |

### Example 3: Transfer of pansy F3'5'H#40 gene and torenia flavone synthase gene into rose variety "Lavande"

A plasmid obtained by inserting the torenia flavone synthase gene reported by Akashi et al. (Plant Cell Physiol 40, 1182-1186, 1999) at the EcoRI and XhoI sites of plasmid pBluescript II SK(-) was designated as pSPB426. After digestion of this plasmid with KpnI, it was blunt ended and further digested with BamHI to obtain an approximately 1.7 kb torenia flavone synthase gene fragment. Separately, pSPB906 described in WO2005/01714 was digested with XhoI and then blunt ended and further digested with BamHI. The torenia flavone synthase gene fragment was inserted between the blunt ends and the BamHI cleavage site to obtain plasmid 906-426.

A plasmid obtained by inserting a fragment of the pansy F3'5'H#40 gene, cut out from pCGP1961 described in WO2005/017147 by partial digestion with BamHI and XhoI, at the BamHI and SalI sites of pSPB176 reported by Ueyama et al. (Ueyama et al. Plant Science, 163, 253-263, 2002), was designated as pSPB575. At the AscI site of this plasmid there was inserted an approximately 3.3 kb torenia flavone synthase gene expression cassette obtained by digesting the aforementioned plasmid 906-426 with AscI. Of the obtained plasmids, the vector having the F3'5'H#40 gene expression cassette and the torenia flavone synthase expression cassette linked in the same direction was designated as pSPB1309. This plasmid constitutively expresses the pansy F3'5'H#40 gene and the torenia flavone synthase gene in plants.

Plasmid pSPB1309 constructed in this manner was transferred into the mauve rose variety "Lavande", and 50 transformants were obtained. Delphinidin accumulation was confirmed in 36 of 38 pigment-analyzed transformants, with a maximum delphinidin content of 45% (average: 12%). Also, novel accumulation of flavones (luteolin and apigenin) was confirmed in 35 transformants, due to the action of the torenia flavone synthase gene. At maximum, the total amount of flavones was a high content of 1.68 mg per 1 g of fresh petal weight.

The analysis values for representative transformants are shown in Table 4 below.

**Table 4**

| Plant No. | Del (%) | Anthocyanidin (mg/g) | | | Flavonol (mg/g) | | | Flavone (mg/g) | | | |
|---|---|---|---|---|---|---|---|---|---|---|---|
| | | Del | Cya | Pel | M | Q | K | Tri | Lut | Api | Total |
| Control | 0.0 | 0.000 | 0.078 | 0.000 | 0.000 | 0.451 | 0.078 | 0.000 | 0.000 | 0.000 | 0.000 |
| 1 | 10.1 | 0.012 | 0.104 | 0.000 | 0.000 | 0.489 | 0.010 | 0.000 | 0.086 | 0.000 | 0.086 |
| 2 | 9.6 | 0.008 | 0.079 | 0.000 | 0.000 | 0.446 | 0.048 | 0.000 | 0.083 | 0.000 | 0.089 |
| 3 | 10.4 | 0.009 | 0.079 | 0.000 | 0.071 | 0.651 | 0. 269 | 0.000 | 0.020 | 0.000 | 0.020 |
| 4 | 44.9 | 0.031 | 0.038 | 0.000 | 0.000 | 0.359 | 0.027 | 0.000 | 1.684 | 0.000 | 1.684 |
| 5 | 33.2 | 0.014 | 0.027 | 0.000 | 0.000 | 0.203 | 0.009 | 0.000 | 1.171 | 0.009 | 1.180 |
| 6 | 37.3 | 0.013 | 0.021 | 0.000 | 0.000 | 0.121 | 0.012 | 0.000 | 0.997 | 0.007 | 1.003 |
| 7 | 39.0 | 0.013 | 0.021 | 0.000 | 0.000 | 0.000 | 0.029 | 0.000 | 1.153 | 0.008 | 1.161 |
| 8 | 35.8 | 0.024 | 0.043 | 0.000 | 0.000 | 0.205 | 0.000 | 0.000 | 1.642 | 0.010 | 1.652 |
| 9 | 36.1 | 0.013 | 0.024 | 0.000 | 0.000 | 1.223 | 0.006 | 0.000 | 0.785 | 0.000 | 0.785 |
| 10 | 32.2 | 0.010 | 0.020 | 0.000 | 0.000 | 0.171 | 0.027 | 0.000 | 0.917 | 0.007 | 0.924 |

| | | | | | | | | | | | |
|---|---|---|---|---|---|---|---|---|---|---|---|
| Control: Lavande control Del: Delphinidin, Cya: Cyanidin, Pel: Pelargonidin, M: Myricetin, Q: Quercetin, K: Kaempferol, Tri: Tricetin, Lut: Luteolin, Api: Apigenin Del(%): Proportion of delphinidin in total anthocyanidins | | | | | | | | | | | |

### Example 4: Transfer of pansy F3'5'H#40 gene and torenia flavone synthase gene into rose variety "WKS124"

Plasmid pSPB1309 described in Example 3 was transferred into the salmon-pink rose variety "WKS124", and 40 transformants were obtained. Delphinidin accumulation was confirmed in 26 of 27 pigment-analyzed transformants, with a maximum delphinidin content of 96% (average: 81%). Also, novel accumulation of flavones (tricetin, luteolin and apigenin) was confirmed in 26 transformants, due to the action of the torenia flavone synthase gene. At maximum, the total amount of flavones was a high content of 4.41 mg per 1 g of fresh petal weight.

The analysis values for representative transformants are shown in Table 5 below.

**Table 5**

| Plant No. | Del (%) | Anthocyanidin (mg/g) | | | Flavonol (mg/g) | | | Flavone (mg/g) | | | |
|---|---|---|---|---|---|---|---|---|---|---|---|
| | | Del | Cya | Pel | M | Q | K | Tri | Lut | Api | Total |
| Control | 0.0 | 0.000 | 0.006 | 0.073 | 0.000 | 0.076 | 3.312 | 0.000 | 0.000 | 0.000 | 0.000 |
| 1 | 84.8 | 0.326 | 0.045 | 0.014 | 0.427 | 0.026 | 0.797 | 0.941 | 0.122 | 0.394 | 1.456 |
| 2 | 86.6 | 0.567 | 0.084 | 0.003 | 0.806 | 0.096 | 0.218 | 2.148 | 1.863 | 0.395 | 4.406 |
| 3 | 82.4 | 0.191 | 0.029 | 0.011 | 0.000 | 0.139 | 0.626 | 1.095 | 0.055 | 0.838 | 1.988 |
| 4 | 83.4 | 0.448 | 0.083 | 0.007 | 0.000 | 0.037 | 0.434 | 1.157 | 0.131 | 0.486 | 1.774 |
| 5 | 80.1 | 0.340 | 0.072 | 0.012 | 0.185 | 0.064 | 0.735 | 0.872 | 0.111 | 0.401 | 1.384 |
| 6 | 83.5 | 0.362 | 0.065 | 0.007 | 0.000 | 0.090 | 0.676 | 1.642 | 0.229 | 0.777 | 2.647 |
| 7 | 88.5 | 0.895 | 0.111 | 0.006 | 0.000 | 0.095 | 0.288 | 1.501 | 0.113 | 0.046 | 1.660 |
| 8 | 87.3 | 0.862 | 0.123 | 0.003 | 0.275 | 0.092 | 0.200 | 1.286 | 0.127 | 0.082 | 1.495 |
| 9 | 89.6 | 0.252 | 0.029 | 0.001 | 0.126 | 0.045 | 0.097 | 2.558 | 0.332 | 0.295 | 3.184 |
| 10 | 81.3 | 0.101 | 0.022 | 0.001 | 0.065 | 0.031 | 0.146 | 1.822 | 0.215 | 0.405 | 2.442 |

| | | | | | | | | | | | |
|---|---|---|---|---|---|---|---|---|---|---|---|
| Control: WKS124 control Del: Delphinidin, Cya: Cyanidin, Pel: Pelargonidin, M: Myricetin, Q: Quercetin, K: Kaempferol, Tri: Tricetin, Lut: Luteolin, Api: Apigenin Del(%) : Proportion of delphinidin in total anthocyanidins | | | | | | | | | | | |

### Example 5: Transfer of pansy F3'5'H#40 gene, torenia flavone synthase gene and torenia anthocyanin methyltransferase gene into rose variety "WKS124"

Plasmid pSPB1309 described in Example 3 was treated with PacI for cleavage at the PacI site present near the linkage point between the torenia flavone synthase expression cassette and the pansy F3'5'H#40 gene expression cassette (more specifically, located near the 3'-end of the D8 terminator of the flavone synthase expression cassette) and at the PacI site in the vector multicloning site, to cut out the pansy F3'5'H#40 gene expression cassette.

Separately, the binary vector pSPB1530 having the torenia methyltransferase gene expression cassette, described in WO2003-062428, was cut with PacI and the aforementioned pansy F3'5'H#40 expression cassette was inserted therein in the same direction as the methyltransferase gene expression cassette. This plasmid was designated as TMT-BP40.

Separately, plasmid pSPB1309 was cleaved with AscI to cut out the torenia flavone synthase expression cassette. This was inserted into the AscI site of TMT-BP40 in the same direction as the previous expression cassettes, and the obtained plasmid was designated as pSFL535. This plasmid constitutively expresses the pansy F3'5'H#40 gene, the torenia methyltransferase gene and the torenia flavone synthase gene in plants.

Plasmid pSFL535 obtained in this manner was transferred into the salmon-pink rose variety "WKS124", and 173 transformants were obtained. Accumulation of malvidin (an anthocyanidin that has been methylated at the 3' and 5' positions of delphinidin) was confirmed in 88 of 98 anthocyanidin-analyzed transformants, and the presence of product indicated that the pansy F3'5'H#40 gene and torenia anthocyanin methyltransferase gene were functioning in the rose petals. The malvidin content was a maximum of 84% (average: 50%).

Also, novel accumulation of flavones (tricetin, luteolin and apigenin) was confirmed in 77 transformants, due to the action of the torenia flavone synthase gene. At maximum, the total amount of flavones was a high content of 4.58 mg per 1 g of fresh petal weight. Methylated tricetin was detected in 51 transformants.

The analysis values for representative transformants are shown in Table 6 below.

**Table 6**

| Plant No. | Del* (%) | Mal (%) | Anthocyanidins (mg/g) | | | | | | Flavonols (mg/g) | | | Flavones (mg/g) | | | |
|---|---|---|---|---|---|---|---|---|---|---|---|---|---|---|---|
| | | | Del | Cya | Pet | Pel | Peo | Mal | M | Q | K | Tri | Lut | Api | Total |
| Control | 0.0 | 0.0 | 0.000 | 0.006 | 0.000 | 0.073 | 0.000 | 0.000 | 0.000 | 0.076 | 3.312 | 0.000 | 0.000 | 0.000 | 0.00 |
| 1 | 97.8 | 65.0 | 0.121 | 0.005 | 0.079 | 0.000 | 0.009 | 0.397 | 0.331 | 0.000 | 0.000 | 2.273 | 0.623 | 0.207 | 3.103 |
| 2 | 96.9 | 81.3 | 0.048 | 0.005 | 0.048 | 0.000 | 0.014 | 0.500 | 0.231 | 0.000 | 0.000 | 3.699 | 0.762 | 0.116 | 4.577 |
| 3 | 96.4 | 83.8 | 0.014 | 0.003 | 0.024 | 0.000 | 0.008 | 0.258 | 0.209 | 0.009 | 0.510 | 1.334 | 0.343 | 0.538 | 2.215 |
| 4 | 87.4 | 77.9 | 0.008 | 0.026 | 0.017 | 0.000 | 0.007 | 0.208 | 0.020 | 0.000 | 0.000 | 3.651 | 0.451 | 0.087 | 4.188 |
| 5 | 93.2 | 79.9 | 0.011 | 0.010 | 0.019 | 0.000 | 0.005 | 0.182 | 0.062 | 0.000 | 0.000 | 3.011 | 0.278 | 0.000 | 3.289 |
| 6 | 93.2 | 61.2 | 0.160 | 0.014 | 0.113 | 0.002 | 0.042 | 0.521 | 0.279 | 0.000 | 0.405 | 1.329 | 0.448 | 0.616 | 2.393 |
| 7 | 90.9 | 63.5 | 0.071 | 0.010 | 0.048 | 0.002 | 0.028 | 0.275 | 0.102 | 0.000 | 0.145 | 0.765 | 0.299 | 0.403 | 1.468 |
| 8 | 95.1 | 64.7 | 0.165 | 0.012 | 0.121 | 0.002 | 0.033 | 0.610 | 0.280 | 0.000 | 0.116 | 1.700 | 0.503 | 0.465 | 2.667 |
| 9 | 86.7 | 67.5 | 0.031 | 0.006 | 0.033 | 0.008 | 0.030 | 0.225 | 0.071 | 0.000 | 0.579 | 1.217 | 0.186 | 0.980 | 2.383 |
| 10 | 93.1 | 72.7 | 0.070 | 0.008 | 0.067 | 0.002 | 0.036 | 0.486 | 0.126 | 0.000 | 0.176 | 1.858 | 0.459 | 0.545 | 2.861 |
| 11 | 85.2 | 60.9 | 0.112 | 0.064 | 0.065 | 0.003 | 0.041 | 0.443 | 0.188 | 0.000 | 0.221 | 1.478 | 0.397 | 0.530 | 2.405 |
| 12 | 93.2 | 67.1 | 0.099 | 0.009 | 0.075 | 0.001 | 0.036 | 0.447 | 0.053 | 0.000 | 0.023 | 1.472 | 0.297 | 0.058 | 1.826 |
| 13 | 89.2 | 64.3 | 0.072 | 0.015 | 0.070 | 0.002 | 0.045 | 0.367 | 0.108 | 0.000 | 0.064 | 1.473 | 0.310 | 0.108 | 1.891 |
| 14 | 90.2 | 63.3 | 0.082 | 0.016 | 0.080 | 0.003 | 0.040 | 0.383 | 0.070 | 0.344 | 0.094 | 1.348 | 0.308 | 0.148 | 1.803 |
| 15 | 87.8 | 64.4 | 0.035 | 0.011 | 0.036 | 0.001 | 0.025 | 0.196 | 0.150 | 0.000 | 0.099 | 1.863 | 0.358 | 0.075 | 2.296 |
| 16 | 92.0 | 70.7 | 0.061 | 0.009 | 0.055 | 0.002 | 0.033 | 0.383 | 0.113 | 0.000 | 0.067 | 2.389 | 0.421 | 0.237 | 3.046 |
| 17 | 91.1 | 65.2 | 0.140 | 0.019 | 0.117 | 0.003 | 0.066 | 0.648 | 0.313 | 0.000 | 0.191 | 2.727 | 0.565 | 0.133 | 3.425 |
| 18 | 90.0 | 63.8 | 0.056 | 0.010 | 0.044 | 0.001 | 0.028 | 0.245 | 0.161 | 0.000 | 0.139 | 0.963 | 0.212 | 0.056 | 1.231 |
| 19 | 89.3 | 68.3 | 0.065 | 0.013 | 0.067 | 0.004 | 0.051 | 0.430 | 0.076 | 0.000 | 0.042 | 2.438 | 0.353 | 0.134 | 2.924 |
| 20 | 89.1 | 63.8 | 0.060 | 0.015 | 0.049 | 0.002 | 0.030 | 0.277 | 0.224 | 0.041 | 0.208 | 1.484 | 0.324 | 0.215 | 2.022 |

| | | | | | | | | | | | | | | | |
|---|---|---|---|---|---|---|---|---|---|---|---|---|---|---|---|
| Control: WKS124 control Del: Delphinidin, Cya: Cyanidin, Pet: Petunidin, Pel: Pelargonidin, Peo: Peonidin, Mal: Malvidin, M: Myricetin, Q: Quercetin, K: Kaempferol, Tri: Tricetin, Lut: Luteolin, Api: Apigenin Del(%): Proportion of delphinidinic pigments (delphinidin, petunidin, malvidin) in total anthocyanins, Mal(%): Proportion of malvidin in total anthocyanidins | | | | | | | | | | | | | | | |

### Example 6: Transfer of pansy F3'5'H#40 gene, torenia flavone synthase gene and torenia anthocyanin methyltransferase gene into rose variety "Lavande"

Plasmid pSFL535 described in Example 5 was transferred into the mauve rose variety "Lavande", and 130 transformants were obtained. Accumulation of malvidin (an anthocyanidin that has been methylated at the 3' and 5' positions of delphinidin) was confirmed in 37 of 118 anthocyanidin-analyzed transformants, and the presence of product indicated that the pansy F3'5'H#40 gene and torenia anthocyanin methyltransferase gene were functioning in the rose petals. The malvidin content was a maximum of 55.6% (average: 20.5%).

Also, novel accumulation of flavones (tricetin, luteolin and apigenin) was confirmed in 78 transformants, due to the action of the torenia flavone synthase gene. At maximum, the total amount of flavones was a high content of 5.11 mg per 1 g of fresh petal weight. In addition, methylated tricetin or luteolin was detected in 20 of the flavone-producing transformants.

The analysis values for representative transformants are shown in Table 7 below.

**Table 7**

| Plant No. | Del (%) | Mal (%) | Anthocyanidins (mg/g) | | | | | | Flavonols (mg/g) | | | Flavones(mg/g) | | | |
|---|---|---|---|---|---|---|---|---|---|---|---|---|---|---|---|
| | | | Del | Cya | Pet | Pel | Peo | Mal | M | Q | K | Tri | Lut | Api | Total |
| Control (Lavande) | 0% | 0% | 0.000 | 0.109 | 0.000 | 0.000 | 0.000 | 0.000 | 0.000 | 1.020 | 0.195 | 0.000 | 0.000 | 0.000 | 0.000 |
| 1 | 20.7% | 3.3% | 0.012 | 0.065 | 0.002 | 0.001 | 0.002 | 0.003 | 0.097 | 0.272 | 0.015 | 0.289 | 0.035 | 0.000 | 0.324 |
| 2 | 45.9% | 31.8% | 0.003 | 0.006 | 0.001 | 0.000 | 0.007 | 0.007 | 0.024 | 0.076 | 0.000 | 1.062 | 0.212 | 0.009 | 1.283 |
| 3 | 74.8% | 46.5% | 0.063 | 0.023 | 0.010 | 0.000 | 0.041 | 0.119 | 0.458 | 0.285 | 0.049 | 0.204 | 0.022 | 0.000 | 0.226 |
| 4 | 71.4% | 51.6% | 0.018 | 0.010 | 0.005 | 0.000 | 0.022 | 0.058 | 0.292 | 0.153 | 0.011 | 0.139 | 0.015 | 0.000 | 0.153 |
| 5 | 70.5% | 32.1% | 0.025 | 0.012 | 0.007 | 0.000 | 0.012 | 0.027 | 0.510 | 0.192 | 0.033 | 0.000 | 0.026 | 0.000 | 0.026 |
| 6 | 28.9% | 4.4% | 0.031 | 0.096 | 0.005 | 0.000 | 0.005 | 0.006 | 0.268 | 0.619 | 0.037 | 0.262 | 0.038 | 0.009 | 0.310 |
| 7 | 84.4% | 53.4% | 0.036 | 0.008 | 0.014 | 0.000 | 0.017 | 0.086 | 0.811 | 0.168 | 0.000 | 1.054 | 0.086 | 0.000 | 1.139 |
| 8 | 79.8% | 53.2% | 0.032 | 0.011 | 0.012 | 0.000 | 0.022 | 0.087 | 0.316 | 0.107 | 0.004 | 0.863 | 0.037 | 0.000 | 0.900 |
| 9 | 83.3% | 55.6% | 0.038 | 0.012 | 0.012 | 0.006 | 0.012 | 0.100 | 0.593 | 0.013 | 0.000 | 4.885 | 0.223 | 0.000 | 5.108 |
| 10 | 63.0% | 33.6% | 0.003 | 0.002 | 0.000 | 0.001 | 0.001 | 0.003 | 0.032 | 0.000 | 0.000 | 3.992 | 0.219 | 0.003 | 4.214 |
| 11 | 88.1% | 45.8% | 0.027 | 0.004 | 0.006 | 0.000 | 0.005 | 0.036 | 0.285 | 0.060 | 0.009 | 2.779 | 0.077 | 0.000 | 2.855 |
| 12 | 86.2% | 43.4% | 0.041 | 0.009 | 0.011 | 0.000 | 0.008 | 0.053 | 0.412 | 0.103 | 0.020 | 4.243 | 0.119 | 0.000 | 4.363 |
| 13 | 73.6% | 38.0% | 0.019 | 0.009 | 0.004 | 0.000 | 0.009 | 0.025 | 0.227 | 0.049 | 0.000 | 3.794 | 0.000 | 0.000 | 3.794 |
| 14 | 89.3% | 49.7% | 0.035 | 0.005 | 0.007 | 0.000 | 0.006 | 0.052 | 0.217 | 0.030 | 0.000 | 4.936 | 0.139 | 0.000 | 5.075 |
| 15 | 65.1% | 31.1% | 0.010 | 0.007 | 0.005 | 0.000 | 0.007 | 0.013 | 0.155 | 0.050 | 0.000 | 3.184 | 0.128 | 0.000 | 3.312 |

| | | | | | | | | | | | | | | | |
|---|---|---|---|---|---|---|---|---|---|---|---|---|---|---|---|
| Control: Lavande control Del: Delphinidin, Cya: Cyanidin, Pet: Petunidin, Pel: Pelargonidin: Peo: Peonidin, Mal: Malvidin, M: Myricetin, Q: Quercetin: K: Kaempferol, Tri: Tricetin, Lut: Luteolin, Api: Apigenin Del(%): Proportion of delphinidinic pigments (delphinidin, petunidin, malvidin) in total anthocyanins Mal(%): Proportion of malvidin in total anthocyanidins | | | | | | | | | | | | | | | |

### Example 7: Transfer of pansy F3'5'H#40 gene, torenia flavone synthase gene and torenia anthocyanin methyltransferase gene into rose variety "WKS82"

Plasmid pSFL535 described in Example 5 was transferred into the mauve rose variety "WKS82", and 250 transformants were obtained. Accumulation of malvidin (an anthocyanidin that has been methylated at the 3' and 5' positions of delphinidin) was confirmed in 110 of 232 anthocyanidin-analyzed transformants, and the presence of product indicated that the pansy F3'5'H#40 gene and torenia anthocyanin methyltransferase gene were functioning in the rose petals. The malvidin content was a maximum of 65.2% (average: 19.7%).

Also, novel accumulation of flavones (tricetin, luteolin and apigenin) was confirmed in 125 transformants, due to the action of the torenia flavone synthase gene. At maximum, the total amount of flavones was a high content of 4.71 mg per 1 g of fresh petal weight. In addition, methylated tricetin or luteolin was detected in 80 of the flavone-producing transformants.

The analysis values for representative transformants are shown in Table 8 below.

**Table 8**

| Plant No. | Del (%) | Mal (%) | Anthocyanidins (mg/g) | | | | | | Flavonols (mg/g) | | | Flavones(mg/g) | | | |
|---|---|---|---|---|---|---|---|---|---|---|---|---|---|---|---|
| | | | Del | Cya | Pet | Pel | Peo | Mal | M | Q | K | Tri | Lut | Api | Total |
| Control (WKS82) | 0% | 0% | 0.000 | 0.124 | 0.000 | 0.000 | 0.000 | 0.000 | 0.000 | 1.598 | 0.081 | 0.000 | 0.000 | 0.000 | 0.000 |
| 1 | 57.5% | 46.1% | 0.003 | 0.006 | 0.003 | 0.000 | 0.018 | 0.026 | 0.494 | 0.750 | 0.064 | 0.764 | 0.000 | 0.000 | 0.764 |
| 2 | 70.5% | 51.0% | 0.007 | 0.005 | 0.004 | 0.000 | 0.011 | 0.028 | 0.564 | 0.384 | 0.055 | 2.977 | 0.199 | 0.000 | 3.176 |
| 3 | 82.1% | 65.2% | 0.006 | 0.004 | 0.005 | 0.000 | 0.008 | 0.042 | 0.800 | 0.536 | 0.115 | 0.534 | 0.000 | 0.000 | 0.534 |
| 4 | 75.3% | 57.5% | 0.004 | 0.003 | 0.003 | 0.000 | 0.008 | 0.024 | 0.387 | 0.288 | 0.074 | 1.808 | 0.160 | 0.000 | 1.968 |
| 5 | 55.2% | 37.6% | 0.005 | 0.009 | 0.004 | 0.000 | 0.015 | 0.020 | 1.054 | 0.806 | 0.038 | 0.114 | 0.000 | 0.000 | 0.114 |
| 6 | 48.8% | 37.8% | 0.004 | 0.006 | 0.002 | 0.000 | 0.021 | 0.020 | 0.700 | 1.319 | 0.148 | 0.034 | 0.000 | 0.000 | 0.034 |
| 7 | 78.4% | 62.8% | 0.007 | 0.003 | 0.004 | 0.000 | 0.011 | 0.042 | 0.577 | 0.266 | 0.015 | 0.302 | 0.022 | 0.000 | 0.324 |
| 8 | 54.6% | 39.1% | 0.006 | 0.009 | 0.003 | 0.000 | 0.018 | 0.023 | 0.571 | 0.774 | 0.045 | 0.172 | 0.028 | 0.000 | 0.200 |
| 9 | 73.5% | 57.3% | 0.009 | 0.004 | 0.004 | 0.000 | 0.016 | 0.044 | 0.866 | 0.511 | 0.031 | 0.104 | 0.000 | 0.000 | 0.104 |
| 10 | 75.9% | 57.5% | 0.005 | 0.002 | 0.002 | 0.000 | 0.007 | 0.022 | 0.882 | 0.498 | 0.151 | 0.038 | 0.000 | 0.000 | 0.038 |
| 11 | 69.3% | 52.9% | 0.007 | 0.006 | 0.005 | 0.000 | 0.016 | 0.038 | 0.825 | 0.411 | 0.029 | 0.095 | 0.000 | 0.000 | 0.095 |
| 12 | 71.4% | 50.2% | 0.013 | 0.007 | 0.006 | 0.000 | 0.020 | 0.046 | 0.721 | 0.459 | 0.022 | 0.075 | 0.004 | 0.000 | 0.080 |
| 13 | 59.8% | 42.2% | 0.016 | 0.014 | 0.009 | 0.000 | 0.044 | 0.062 | 1.540 | 1.415 | 0.202 | 0.193 | 0.000 | 0.000 | 0.095 |
| 14 | 67.9% | 50.9% | 0.006 | 0.006 | 0.006 | 0.000 | 0.017 | 0.036 | 0.829 | 0.704 | 0.125 | 0.000 | 0.000 | 0.000 | 0.200 |
| 15 | 34.4% | 13.0% | 0.006 | 0.014 | 0.003 | 0.000 | 0.013 | 0.006 | 0.230 | 1.109 | 0.000 | 4.155 | 0.551 | 0.006 | 4.711 |

| | | | | | | | | | | | | | | | |
|---|---|---|---|---|---|---|---|---|---|---|---|---|---|---|---|
| Control: WKS82 control Del: Delphinidin, Cya: Cyanidin, Pet: Petunidin, Pel: Pelargonidin: Peo: Peonidin, Mal: Malvidin, M: Myricetin, Q: Quercetin: K: Kaempferol, Tri: Tricetin, Lut: Luteolin, Api: Apigenin Del(%): Proportion of delphinidinic pigments (delphinidin, petunidin, malvidin) in total anthocyanins Mal(%): Proportion of malvidin in total anthocyanidins | | | | | | | | | | | | | | | |

### Example 8: Transfer of pansy F3'5'H#40 gene, torenia flavone synthase gene and torenia anthocyanin methyltransferase gene into rose variety "WKS140"

Plasmid pSFL535 described in Example 5 was transferred into the mauve rose variety "WKS140", and 74 transformants were obtained. Accumulation of malvidin (an anthocyanidin that has been methylated at the 3' and 5' positions of delphinidin) was confirmed in 20 of 74 anthocyanidin-analyzed transformants, and the presence of product indicated that the pansy F3'5'H#40 gene and torenia anthocyanin methyltransferase gene were functioning in the rose petals. The malvidin content was a maximum of 51.3% (average: 33.5%).

Also, novel accumulation of flavones (tricetin, luteolin and apigenin) was confirmed in 29 transformants, due to the action of the torenia flavone synthase gene. At maximum, the total amount of flavones was a high content of 3.04 mg per 1 g of fresh petal weight. In addition, methylated tricetin or luteolin was detected in 20 of the flavone-producing transformants.

The analysis values for representative transformants are shown in Table 9 below.

**Table 9**

| Plant No. | Del (%) | Mal (%) | Anthocyanidins (mg/g) | | | | | | Flavonols (mg/g) | | | Flavones (mg/g) | | | |
|---|---|---|---|---|---|---|---|---|---|---|---|---|---|---|---|
| | | | Del | Cya | Pet | Pel | Peo | Mal | M | Q | K | Tri | Lut | Api | Total |
| Control (WKS140) | 0% | 0% | 0.000 | 0.075 | 0.000 | 0.000 | 0.000 | 0.000 | 0.000 | 2.412 | 0.271 | 0.000 | 0.000 | 0.000 | 0.000 |
| 1 | 62.0% | 31.7% | 0.025 | 0.020 | 0.015 | 0.000 | 0.030 | 0.042 | 0.655 | 1.085 | 0.202 | 2.314 | 0.305 | 0.032 | 2.650 |
| 2 | 67.3% | 38.3% | 0.013 | 0.009 | 0.009 | 0.000 | 0.015 | 0.029 | 0.491 | 0.627 | 0.104 | 1.790 | 0.227 | 0.031 | 2.048 |
| 3 | 79.6% | 34.1% | 0.025 | 0.008 | 0.011 | 0.000 | 0.008 | 0.027 | 0.572 | 0.555 | 0.129 | 2.388 | 0.237 | 0.015 | 2.639 |
| 4 | 69.8% | 38.9% | 0.021 | 0.012 | 0.011 | 0.000 | 0.019 | 0.040 | 0.589 | 0.766 | 0.165 | 1.941 | 0.282 | 0.014 | 2.237 |
| 5 | 80.4% | 51.3% | 0.013 | 0.005 | 0.009 | 0.000 | 0.010 | 0.038 | 0.513 | 0.307 | 0.074 | 1.392 | 0.166 | 0.018 | 1.577 |
| 6 | 70.1% | 35.8% | 0.014 | 0.008 | 0.006 | 0.000 | 0.010 | 0.021 | 0.607 | 0.538 | 0.108 | 1.297 | 0.177 | 0.019 | 1.493 |
| 7 | 67.2% | 34.7% | 0.020 | 0.013 | 0.009 | 0.000 | 0.017 | 0.031 | 1.005 | 0.717 | 0.127 | 1.805 | 0.264 | 0.028 | 2.097 |
| 8 | 70.0% | 36.2% | 0.019 | 0.010 | 0.009 | 0.000 | 0.015 | 0.029 | 0.831 | 0.802 | 0.143 | 1.909 | 0.241 | 0.027 | 2.176 |
| 9 | 70.9% | 37.9% | 0.015 | 0.008 | 0.008 | 0.000 | 0.012 | 0.027 | 0.497 | 0.690 | 0.106 | 1.841 | 0.265 | 0.032 | 2.138 |
| 10 | 69.9% | 36.0% | 0.018 | 0.010 | 0.009 | 0.000 | 0.015 | 0.030 | 0.544 | 0.663 | 0.143 | 2.102 | 0.236 | 0.017 | 2.355 |
| 11 | 57.4% | 31.0% | 0.011 | 0.011 | 0.008 | 0.000 | 0.019 | 0.022 | 0.386 | 0.892 | 0.129 | 2.088 | 0.271 | 0.012 | 2.372 |
| 12 | 62.9% | 32.4% | 0.016 | 0.014 | 0.010 | 0.000 | 0.018 | 0.028 | 0.351 | 0.846 | 0.114 | 2.274 | 0.281 | 0.009 | 2.565 |
| 13 | 62.1% | 34.1% | 0.021 | 0.018 | 0.014 | 0.000 | 0.030 | 0.042 | 0.887 | 0.789 | 0.177 | 1.855 | 0.389 | 0.018 | 2.262 |
| 14 | 73.7% | 37.5% | 0.016 | 0.006 | 0.004 | 0.000 | 0.008 | 0.021 | 0.597 | 0.489 | 0.081 | 1.664 | 0.158 | 0.000 | 1.821 |
| 15 | 58.6% | 28.3% | 0.013 | 0.012 | 0.007 | 0.000 | 0.016 | 0.019 | 0.513 | 1.121 | 0.166 | 2.650 | 0.373 | 0.015 | 3.038 |

| | | | | | | | | | | | | | | | |
|---|---|---|---|---|---|---|---|---|---|---|---|---|---|---|---|
| Control: WKS140 control Del: Delphinidin, Cya: Cyanidin, Pet: Petunidin, Pel: Pelargonidin: Peo: Peonidin, Mal: Malvidin, M: Myricetin, Q: Quercetin: K: Kaempferol, Tri: Tricetin, Lut: Luteolin, Api: Apigenin Del(%): Proportion of delphinidinic pigments (delphinidin, petunidin, malvidin) in total anthocyanins Mal(%): Proportion of malvidin in total anthocyanidins | | | | | | | | | | | | | | | |

### Example 9: Propagation of flavone and malvidin synthesis ability to progeny - Hybridization between cultivated roses and gene recombinant roses containing transferred pansy F3'5'H#40 gene, torenia flavone synthase gene and torenia anthocyanin methyltransferase gene

In order to investigate the mode of inheritance to progeny for flavone synthesis ability in roses, cross-breeding was carried out using a malvidin- and flavone-producing rose created in Example 5 (plant No. 6 in Table 6) as the pollen parent. As the seed parent there was used the medium-sized cultivated rose "Medeo" (floribunda rose variety "Medeo").

Accumulation of malvidin was confirmed in 7 of the 10 pigment-analyzed transformant F1 hybrid progeny that were obtained, and the presence of product indicated that the pansy F3'5'H#40 gene and torenia anthocyanin methyltransferase gene were functioning in the rose petals. The malvidin content was a maximum of 68.2% (average: 46.6%).

On the other hand, novel accumulation of flavones (tricetin, luteolin and apigenin) was confirmed in 8 transformant progeny, due to the action of the torenia flavone synthase gene. At maximum, the total amount of flavones was an extremely high content of 7.35 mg per 1 g of fresh petal weight. In addition, methylated tricetin or luteolin was detected in 6 of the flavone-producing transformant progeny.

The analysis values for representative transformant progeny are shown in Table 10 below.

**Table 10**

| Plant No. | Del (%) | Mal (%) | Anthocyanidins (mg/g) | | | | | | Flavonols (mg/g) | | | Flavones(mg/g) | | | |
|---|---|---|---|---|---|---|---|---|---|---|---|---|---|---|---|
| | | | Del | Cya | Pet | Pel | Peo | Mal | M | Q | K | Tri | Lut | Api | Total |
| Pollen parent (Example 5 Plant No.6) | 93.2% | 61.2% | 0.160 | 0.014 | 0.113 | 0.002 | 0.042 | 0.521 | 0.279 | 0.000 | 0.405 | 1.329 | 0.448 | 0.616 | 2.393 |
| Seed parent (var. Medeo) | 0% | 0% | 0.000 | 0.004 | 0.000 | 0.004 | 0.000 | 0.000 | 0.000 | 0.028 | 2.323 | 0.000 | 0.000 | 0.000 | 0.000 |
| 1 | 0.0% | 0.0% | 0.000 | 0.015 | 0.000 | 0.122 | 0.000 | 0.000 | 0.000 | 0.000 | 4.318 | 0.000 | 0.000 | 0.000 | 0.000 |
| 2 | 82.6% | 49.1% | 0.165 | 0.034 | 0.085 | 0.005 | 0.090 | 0.367 | 0.311 | 0.039 | 0.118 | 1.596 | 0.064 | 0.006 | 1.666 |
| 3 | 0.0% | 0.0% | 0.000 | 0.001 | 0.000 | 0.004 | 0.000 | 0.000 | 0.000 | 0.000 | 2.391 | 0.000 | 0.000 | 0.000 | 0.000 |
| 4 | 80.1% | 50.5% | 0.073 | 0.028 | 0.064 | 0.00 | 0.054 | 0.233 | 0.210 | 0.048 | 0.429 | 0.000 | 0.000 | 0.032 | 0.032 |
| 5 | 94.4% | 52.8% | 0.003 | 0.001 | 0.003 | 0.000 | 0.000 | 0.009 | 0.408 | 0.069 | 0.668 | 2.024 | 0.152 | 0.222 | 2.398 |
| 6 | 81.8% | 34.4% | 0.056 | 0.015 | 0.034 | 0.002 | 0.017 | 0.065 | 0.076 | 0.033 | 0.202 | 3.860 | 0.123 | 0.039 | 4.023 |
| 7 | 48.2% | 0.0% | 0.011 | 0.002 | 0.011 | 0.001 | 0.021 | 0.000 | 0.089 | 0.038 | 0.808 | 1.603 | 0.117 | 0.217 | 1.937 |
| 8 | 90.6% | 35.5% | 0.107 | 0.016 | 0.071 | 0.002 | 0.013 | 0.114 | 0.080 | 0.010 | 0.100 | 6.497 | 0.351 | 0.504 | 7.351 |
| 9 | 70.4% | 35.8% | 0.008 | 0.003 | 0.001 | 0.002 | 0.003 | 0.009 | 0.118 | 0.038 | 0.523 | 2.902 | 0.137 | 0.048 | 3.088 |
| 10 | 91.2% | 68.2% | 0.011 | 0.002 | 0.012 | 0.001 | 0.007 | 0.068 | 1.131 | 0.324 | 1.077 | 1.031 | 0.091 | 0.033 | 1.154 |

| | | | | | | | | | | | | | | | |
|---|---|---|---|---|---|---|---|---|---|---|---|---|---|---|---|
| Del: Delphinidin, Cya: Cyanidin, Pet: Petunidin, Pel: Pelargonidin: Peo: Peonidin, Mal: Malvidin, M: Myricetin, Q: Quercetin: K: Kaempferol, Tri: Tricetin, Lut: Luteolin, Api: Apigenin Del(%): Proportion of delphinidinic pigments (delphinidin, petunidin, malvidin) in total anthocyanins Mal(%): Proportion of malvidin in total anthocyanidins | | | | | | | | | | | | | | | |

### Example 10: Propagation of flavone synthesis ability to progeny

### Cross-breeding of rose variety "WKS124" containing transferred pansy F3'5'H#40 gene and torenia anthocyanin methyltransferase gene, with rose variety "Lavande" containing transferred pansy F3'5'H#40 gene and torenia flavone synthase gene.

In order to investigate the mode of inheritance to progeny for flavone synthesis ability in roses, cross-breeding was carried out using a flavone-producing line created in Example 3 (plant No. 4 in Table 4) as the pollen parent. As the seed parent there was used transformant WKS124/1532-12-1 (described in WO2003/062428), with high accumulation of malvidin in the petals due to transfer of pSPB1532 into the rose variety WKS124 and the resulting actions of the pansy F3'5'H#40 gene and torenia anthocyanin methyltransferase gene.

Upon pigment analysis of 149 of the obtained transformant progeny, accumulation of flavones (tricetin, luteolin, apigenin) was confirmed in 88 individuals. At maximum, the total amount of flavones was a high content of 4.09 mg per 1 g of fresh petal weight. Also, methylated tricetin was detected in 42 progeny, while methylated luteolin (chrysoeriol (3'-Met-Lut)) was detected in 11. Accumulation of malvidin was confirmed in 129 of the 149 pigment-analyzed progeny. The malvidin content was a maximum of 79% (average: 36%).

The analysis values for representative transformant progeny are shown in Table 11 below.

**Table 11**

| Plant No. | Del* (%) | Mal (%) | Anthocyanidins (mg/g) | | | | | | Flavonols (mg/g) | | | Flavones (mg/g) | | | |
|---|---|---|---|---|---|---|---|---|---|---|---|---|---|---|---|
| | | | Del | Cya | Pet | Pel | Peo | Mal | M | Q | K | Tri | Lut | Api | Total |
| Pollen parent | 44.9 | 0.0 | 0.031 | 0.038 | 0.000 | 0.000 | 0.000 | 0.000 | 0.000 | 0.359 | 0.027 | 0.000 | 1.684 | 0.000 | 1.684 |
| Seed parent | 93.2 | 73.0 | 0.127 | 0.011 | 0.112 | 0.003 | 0.066 | 0.863 | 0.365 | 0.093 | 0.348 | 0.000 | 0.000 | 0.000 | 0.000 |
| 1 | 92.1 | 69.1 | 0.032 | 0.005 | 0.030 | 0.000 | 0.016 | 0.186 | 0.197 | 0.105 | 0.090 | 1.950 | 0.078 | 0.059 | 2.088 |
| 2 | 75.3 | 56.7 | 0.076 | 0.048 | 0.055 | 0.005 | 0.121 | 0.400 | 0.345 | 0.081 | 0.097 | 2.879 | 0.156 | 0.086 | 3.121 |
| 3 | 80.6 | 60.6 | 0.041 | 0.015 | 0.039 | 0.004 | 0.059 | 0.244 | 0.000 | 0.000 | 0.113 | 2.986 | 0.193 | 0.000 | 3.179 |
| 4 | 82.4 | 65.8 | 0.005 | 0.002 | 0.006 | 0.000 | 0.009 | 0.043 | 0.000 | 0.131 | 0.084 | 2.036 | 0.066 | 0.000 | 2.103 |
| 5 | 68.8 | 56.7 | 0.010 | 0.006 | 0.012 | 0.013 | 0.036 | 0.101 | 0.000 | 0.093 | 0.179 | 1.740 | 0.224 | 0.000 | 1.965 |
| 6 | 79.3 | 60.4 | 0.018 | 0.009 | 0.017 | 0.000 | 0.029 | 0.111 | 0.089 | 0.053 | 0.084 | 1.956 | 0.093 | 0.029 | 2.078 |
| 7 | 86.1% | 52.1 | 0.158 | 0.044 | 0.125 | 0.003 | 0.069 | 0.432 | 0.000 | 0.118 | 0.300 | 3.059 | 0.363 | 0.397 | 3.819 |
| 8 | 81.3 | 59.8 | 0.026 | 0.010 | 0.027 | 0.011 | 0.025 | 0.149 | 0.000 | 0.247 | 0.226 | 1.489 | 0.232 | 0.179 | 1.900 |
| 9 | 79.2 | 59.2% | 0.015 | 0.008 | 0.014 | 0.000 | 0.022 | 0.086 | 0.422 | 0.398 | 0.224 | 2.510 | 0.726 | 0.094 | 3.330 |
| 10 | 82.5 | 66.8% | 0.019 | 0.008 | 0.022 | 0.000 | 0.038 | 0.175 | 0.445 | 0.559 | 0.322 | 2.122 | 0.446 | 0.111 | 2.678 |
| 11 | 73.3 | 59.3 | 0.036 | 0.025 | 0.037 | 0.000 | 0.112 | 0.306 | 0.121 | 0.130 | 0.000 | 0.596 | 0.565 | 0.066 | 1.227 |
| 12 | 94.0 | 76.2 | 0.018 | 0.002 | 0.018 | 0.000 | 0.010 | 0.154 | 0.840 | 0.426 | 0.445 | 3.655 | 0.306 | 0.124 | 4.086 |
| 13 | 82.8 | 62.8 | 0.009 | 0.004 | 0.010 | 0.000 | 0.012 | 0.059 | 0.394 | 0.400 | 0.278 | 1.068 | 0.250 | 0.096 | 1.414 |
| 14 | 82.7 | 58.8 | 0.119 | 0.048 | 0.122 | 0.07.1 | 0.115 | 0.592 | 0.327 | 0.089 | 0.074 | 1.940 | 0.131 | 0.085 | 2.156 |
| 15 | 78.4 | 61.6 | 0.009 | 0.006 | 0.009 | 0.000 | 0.018 | 0.066 | 0.313 | 0.582 | 0.370 | 1.634 | 0.423 | 0.143 | 2.200 |

| | | | | | | | | | | | | | | | |
|---|---|---|---|---|---|---|---|---|---|---|---|---|---|---|---|
| Del: Delphinidin, Cya: Cyanidin, Pet: Petunidin, Pel: Pelargonidin, Peo: Peonidin, Mal: Malvidin, M: Myricetin, Q: Quercetin, K: Kaempferol, Tri: Tricetin, Lut: Luteolin, Api: Apigenin Del(%): Proportion of delphinidinic pigments (delphinidin, petunidin, malvidin) in total anthocyanins Mal(%): Proportion of malvidin in total anthocyanidins | | | | | | | | | | | | | | | |

### Example 11: Evaluation of flavone-containing rose flower color

The transformants created in Examples 4 and 5 (host: rose variety "WKS124") were divided into 5 groups: (1) those accumulating delphinidin as the major pigment and containing no flavones, (2) those accumulating delphinidin as the major pigment and containing flavones, (3) those highly accumulating malvidin as the major pigment and containing no flavones, (4) those highly accumulating malvidin as the major pigment and containing flavones, and (5) host (accumulating pelargonidin as the major pigment), and the color shade of the petals were evaluated using a spectrocolorimeter (n = 10).

In both the roses with delphinidin as the major pigment and the roses with malvidin as the major pigment, a shift in hue angle of the petals toward blue had occurred when flavones were copresent. This tendency was more pronounced in the roses with malvidin as the major pigment, and the reflection spectrum minimum (λMin) was also shifted significantly toward the long wavelength end. These results confirmed that the petal color shade had changed to blue by the copresence of flavones. The results are shown in Table 12 below.

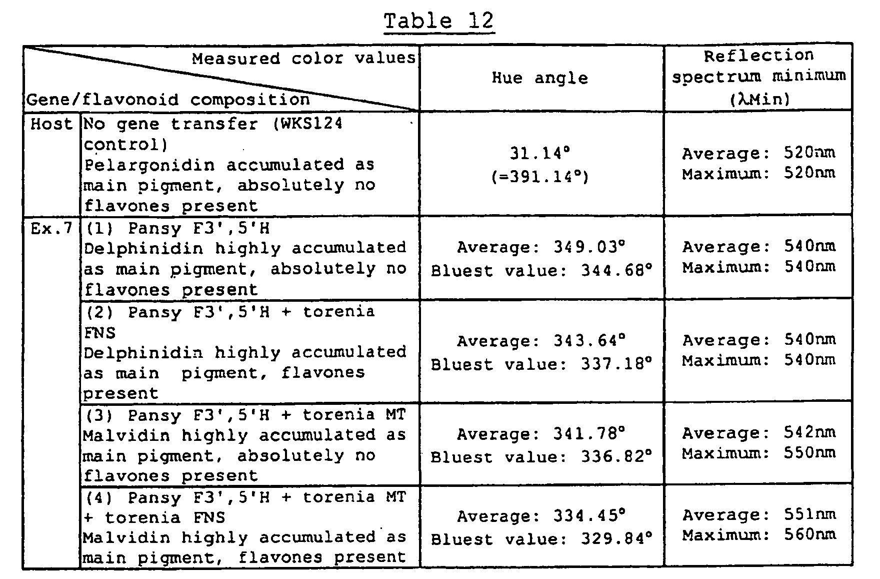

Hue angle (hue): The angle displacement for the color tone in the counter-clockwise direction from the a* (red direction) axis as 0° in the L*a*b* color system, for indication of the color position. An angle of 90° is the yellow direction, an angle of 180° is the green direction, an angle of 270° is the blue direction, and an angle of 0° (= 360°) is the red direction. In other words, a numerical value approaching 270° represents a bluer color tone.

### Industrial Applicability

According to the invention it is possible by genetic modification to add flavones and malvidin to roses, as popular flowering plants used for decoration, in order to alter rose flower color toward blue by a co-pigmentation effect. Roses with blue flower color are expected to be in high commercial demand as ornamental plants.

This completes the explanation of the invention, but the invention should be interpreted as encompassing any alterations or modifications such as do not deviate from the gist thereof, and the scope of the invention is not to be considered as based on the description in the examples but rather as defined by the scope of the attached claims.

### SEQUENCE LISTING

<110> International Flower Developments Proprietary Limited
   <120> Rose containing flavone and malvidin and method for production thereof
   <130> R3121 EP
   <140> EP 08 77 7606.8
   <141> 2008-06-19
   <150> JP 2007-162959
   <151> 2007-06-20
   <160> 10
   <210> 1
   <212> 1706
   <212> DNA
   <213> Antirrhinum majus
   <220>
   <221> CDS
   <222> (41)...(1557)
   <223> Nucleotide sequence encoding flavone synthase
   <400> 1 
gctttacaca cacacacaca cacacacaca caaacaaaa atg tct aca ctt gtc tac     57
                                          Met Ser Thr Leu Val Tyr
                                           1               5
agc aca ctc ttc atc ctc tca acc ctc ctc ctc acc ctc cta acc cgc     105
Ser Thr Leu Phe Ile Leu Ser Thr Leu Leu Leu Thr Leu Leu Thr Arg
            10                  15                  20
acc cgc cgc aag acc cgc ccg ccc ggc cca tta gcc ctc ccc tta ata     153
Thr Arg Arg Lys Thr Arg Pro Pro Gly Pro Leu Ala Leu Pro Leu Ile
        25                  30                  35
ggc cac tta cac ctc ctc ggc cca aag ctc cac cac acc ttc cac caa     201
Gly His Leu His Leu Leu Gly Pro Lys Leu His His Thr Phe His Gln
    40                  45                  50
ttc tcc caa cgc tac ggc ccg ctc atc cag ctc tac ctc ggc tcc gtc     249
Phe Ser Gln Arg Tyr Gly Pro Leu Ile Gln Leu Tyr Leu Gly Ser Val
55                  60                  65                  70
cca tgc gtc gtc gct tcc acg ccc gaa ctc gcc cgc gaa ttc ctc aag     297
Pro Cys Val Val Ala Ser Thr Pro Glu Leu Ala Arg Glu Phe Leu Lys
                75                  80                  85
acg cac gaa ctc gac ttc tcg tcc cgc aag cac tcc acc gcc atc gac     345
Thr His Glu Leu Asp Phe Ser Ser Arg Lys His Ser Thr Ala Ile Asp
            90                  95                  100
atc gtc acg tac gac tcc tcg ttc gcc ttc gcg ccg tac ggg ccg tac     393
Ile Val Thr Tyr Asp Ser Ser Phe Ala Phe Ala Pro Tyr Gly Pro Tyr
        105                 110                 115
tgg aaa ttc atc aag aaa tta tgt act tac gag cta ctg ggt gcc cgg     441
Trp Lys Phe Ile Lys Lys Leu Cys Thr Tyr Glu Leu Leu Gly Ala Arg
    120                 125                 130
aac ttg agc cat ttc cag ccc att aga gct ttg gag gtc aac agt ttc     489
Asn Leu Ser His Phe Gln Pro Ile Arg Ala Leu Glu Val Asn Ser Phe
135                 140                 145                 150
ttg aga att ttg tac gag aaa aca gag cag aaa cag agt gtt aat gtg     537
Leu Arg Ile Leu Tyr Glu Lys Thr Glu Gln Lys Gln Ser Val Asn Val
                155                 160                 165
act gag gag ctt gtg aag ctg acg agt aat gtg atc agt aac atg atg     585
Thr Glu Glu Leu Val Lys Leu Thr Ser Asn Val Ile Ser Asn Met Met
            170                 175                 180
ttg ggg atc agg tgt tcg ggg acg gaa ggg gag gcg gag gtg gcg agg     633
Leu Gly Ile Arg Cys Ser Gly Thr Glu Gly Glu Ala Glu Val Ala Arg
        185                 190                 195
acg gtg ata agg gag gtg acg cag ata ttt ggg gag ttt gat gtg tcg     681
Thr Val Ile Arg Glu Val Thr Gln Ile Phe Gly Glu Phe Asp Val Ser
 
200                 205                 210
gag att gtt tgg ttt tgt aag aat ttg gat ctg cag ggg att agg aag      729
Glu Ile Val Trp Phe Cys Lys Asn Leu Asp Leu Gln Gly Ile Arg Lys
215                 220                 225                 230
agg tcg gag gat att agg agg agg tat gat gct ttg ttg gag aag att      777
Arg Ser Glu Asp Ile Arg Arg Arg Tyr Asp Ala Leu Leu Glu Lys Ile
                235                 240                 245
att agt gat agg gag agg ttg agg ttg agg ggg ggt ggt ggt gga ggg      825
Ile Ser Asp Arg Glu Arg Leu Arg Leu Arg Gly Gly Gly Gly Gly Gly
            250                 255                 260
ggt gga gag gtg aag gat ttt ttg gat atg ttg ttg gat gtg atg gag      873
Gly Gly Glu Val Lys Asp Phe Leu Asp Met Leu Leu Asp Val Met Glu
        265                 270                 275
agt gag aaa tcg gag gtg gag ttt acg agg gag cat ctc aaa gct ttg      921
Ser Glu Lys Ser Glu Val Glu Phe Thr Arg Glu His Leu Lys Ala Leu
    280                 285                 290
att ctg gat ttc ttc act gcc ggt aca gac aca aca gca atc aca aca      969
Ile Leu Asp Phe Phe Thr Ala Gly Thr Asp Thr Thr Ala Ile Thr Thr
295                 300                 305                 310
gaa tgg gca ata gca gaa ctc att agc aat cca aat gta ctc aaa aaa     1017
Glu Trp Ala Ile Ala Glu Leu Ile Ser Asn Pro Asn Val Leu Lys Lys
                315                 320                 325
gct caa gaa gag atg gac aaa gtc ata gga tca caa agg ttg ttg caa     1065
Ala Gln Glu Glu Met Asp Lys Val Ile Gly Ser Gln Arg Leu Leu Gln
            330                 335                 340
gaa tcc gac gcc cct aac ttg cct tac ctc aac gcg atc ata aaa gaa     1113
Glu Ser Asp Ala Pro Asn Leu Pro Tyr Leu Asn Ala Ile Ile Lys Glu
        345                 350                 355
acg ttc cgt ctc cac cct cca atc ccc atg ctc act aga aaa tca att     1161
Thr Phe Arg Leu His Pro Pro Ile Pro Met Leu Thr Arg Lys Ser Ile
    360                 365                 370
tct gac gtt gtg gtc aac ggg tac acg atc cct gcc aaa acg cta ttg     1209
Ser Asp Val Val Val Asn Gly Tyr Thr Ile Pro Ala Lys Thr Leu Leu
375                 380                 385                 390
ttt gtc aac ctt tgg tcc atg gga agg aat cct aac tac tgg gaa aat     1257
Phe Val Asn Leu Trp Ser Met Gly Arg Asn Pro Asn Tyr Trp Glu Asn
                395                 400                 405
ccg atg gag ttc cga ccc gag agg ttt ctc gag aaa ggg acc ggg tcg     1305
Pro Met Glu Phe Arg Pro Glu Arg Phe Leu Glu Lys Gly Thr Gly Ser
            410                 415                 420
ata gac gtt aaa ggg cag cat ttc gag ttg ctg ccg ttt ggc acg ggc     1353
Ile Asp Val Lys Gly Gln His Phe Glu Leu Leu Pro Phe Gly Thr Gly
        425                 430                 435
agg cgg ggc tgc ccg ggg atg ttg tta ggc atg cag gag ttg ttt agt     1401
Arg Arg Gly Cys Pro Gly Met Leu Leu Gly Met Gln Glu Leu Phe Ser
    440                 445                 450
att atc ggg gct atg gtg cag tgc ttc gat tgg aaa ctg ccc gat ggt     1449
Ile Ile Gly Ala Met Val Gln Cys Phe Asp Trp Lys Leu Pro Asp Gly
455                 460                 465                 470
gtg aag tcg gtc gac atg acc gag cgg ccc ggg ttg acg gct cca cgt     1497
Val Lys Ser Val Asp Met Thr Glu Arg Pro Gly Leu Thr Ala Pro Arg
                475                 480                 485
gcc aat gat ttg gtg tgc caa ttg gtg cca cgg att gac ccg gtc gtt     1545
Ala Asn Asp Leu Val Cys Gln Leu Val Pro Arg Ile Asp Pro Val Val
            490                 495                 500
gtc tcc gga ccg tgaaccttaa ggtagtatcg ataatctgtt taattaaatt         1597
Val Ser Gly Pro
        505
gttatttgtt ctgaggattt gatttttgtt atgtatgatt atgcgtggat taagataagc   1657
ctgcaaggac aaattccctt tctttgattg atgtcaatga gtttgtgtc               1706
 <210> 2
   <211> 506
   <212> PRT
   <213> Antirrhinum majus
   <220>
   <221>
   <222>
   <223> Amino acid sequence of flavone synthase
   <400> 2 
Met Ser Thr Leu Val Tyr Ser Thr Leu Phe Ile Leu Ser Thr Leu Leu
1               5                   10                  15
Leu Thr Leu Leu Thr Arg Thr Arg Arg Lys Thr Arg Pro Pro Gly Pro
            20                  25                  30
Leu Ala Leu Pro Leu Ile Gly His Leu His Leu Leu Gly Pro Lys Leu
        35                  40                  45
His His Thr Phe His Gln Phe Ser Gln Arg Tyr Gly Pro Leu Ile Gln
    50                  55                  60
Leu Tyr Leu Gly Ser Val Pro Cys Val Val Ala Ser Thr Pro Glu Leu
65                  70                  75                  80
Ala Arg Glu Phe Leu Lys Thr His Glu Leu Asp Phe Ser Ser Arg Lys
                85                  90                  95
His Ser Thr Ala Ile Asp Ile Val Thr Tyr Asp Ser Ser Phe Ala Phe
            100                 105                 110
Ala Pro Tyr Gly Pro Tyr Trp Lys Phe Ile Lys Lys Leu Cys Thr Tyr
        115                 120                 125
Glu Leu Leu Gly Ala Arg Asn Leu Ser His Phe Gln Pro Ile Arg Ala
    130                 135                 140
Leu Glu Val Asn Ser Phe Leu Arg Ile Leu Tyr Glu Lys Thr Glu Gln
145                 150                 155                 160
Lys Gln Ser Val Asn Val Thr Glu Glu Leu Val Lys Leu Thr Ser Asn
                165                 170                 175
Val Ile Ser Asn Met Met Leu Gly Ile Arg Cys Ser Gly Thr Glu Gly
            180                 185                 190
Glu Ala Glu Val Ala Arg Thr Val Ile Arg Glu Val Thr Gln Ile Phe
        195                 200                 205
Gly Glu Phe Asp Val Ser Glu Ile Val Trp Phe Cys Lys Asn Leu Asp
    210                 215                 220
Leu Gln Gly Ile Arg Lys Arg Ser Glu Asp Ile Arg Arg Arg Tyr Asp
225                 230                 235                 240
Ala Leu Leu Glu Lys Ile Ile Ser Asp Arg Glu Arg Leu Arg Leu Arg
                245                 250                 255
Gly Gly Gly Gly Gly Gly Gly Gly Glu Val Lys Asp Phe Leu Asp Met
            260                 265                 270
Leu Leu Asp Val Met Glu Ser Glu Lys Ser Glu Val Glu Phe Thr Arg
        275                 280                 285
Glu His Leu Lys Ala Leu Ile Leu Asp Phe Phe Thr Ala Gly Thr Asp
    290                 295                 300
Thr Thr Ala Ile Thr Thr Glu Trp Ala Ile Ala Glu Leu Ile Ser Asn
305                 310                 315                 320
Pro Asn Val Leu Lys Lys Ala Gln Glu Glu Met Asp Lys Val Ile Gly
                325                 330                 335
Ser Gln Arg Leu Leu Gln Glu Ser Asp Ala Pro Asn Leu Pro Tyr Leu
            340                 345                 350
Asn Ala Ile Ile Lys Glu Thr Phe Arg Leu His Pro Pro Ile Pro Met
        355                 360                 365
Leu Thr Arg Lys Ser Ile Ser Asp Val Val Val Asn Gly Tyr Thr Ile
    370                 375                 380
Pro Ala Lys Thr Leu Leu Phe Val Asn Leu Trp Ser Met Gly Arg Asn
385                 390                 395                 400
Pro Asn Tyr Trp Glu Asn Pro Met Glu Phe Arg Pro Glu Arg Phe Leu
 
                405                 410                 415
Glu Lys Gly Thr Gly Ser Ile Asp Val Lys Gly Gln His Phe Glu Leu
            420                 425                 430
Leu Pro Phe Gly Thr Gly Arg Arg Gly Cys Pro Gly Met Leu Leu Gly
        435                 440                 445
Met Gln Glu Leu Phe Ser Ile Ile Gly Ala Met Val Gln Cys Phe Asp
    450                 455                 460
Trp Lys Leu Pro Asp Gly Val Lys Ser Val Asp Met Thr Glu Arg Pro
465                 470                 475                 480
Gly Leu Thr Ala Pro Arg Ala Asn Asp Leu Val Cys Gln Leu Val Pro
                485                 490                 495
Arg Ile Asp Pro Val Val Val Ser Gly Pro
            500                 505
 <210> 3
   <211> 1685
   <212> DNA
   <213> Torenia hybrida
   <220>
   <221> CDS
   <222> (57)...(1592)
   <223> Nucleotide sequence encoding flavone synthase
   <400> 3 
atcgaaaccg ctatatcatt acatttacaa cagcgctaaa aaaatatata taaagc atg 59
Met
1

gac aca gtc tta atc aca ctc tac acc gcc ctg ttc gtc atc acc acc 107
Asp Thr Val Leu Ile Thr Leu Tyr Thr Ala Leu Phe Val Ile Thr Thr
            5                   10                  15

acc ttc ctc ctc ctc ctc cgc cga agg gga cca ccg tct ccg ccc ggt 155
Thr Phe Leu Leu Leu Leu Arg Arg Arg Gly Pro Pro Ser Pro Pro Gly
        20                  25                  30

cct ctc tcc cta ccc ata att ggc cac ctc cac ctc ctc ggc cca aga 203
Pro Leu Ser Leu Pro Ile Ile Gly His Leu His Leu Leu Gly Pro Arg
    35                  40                  45

ctc cac cac acg ttc cat gaa ttc tca ctc aaa tac ggc cca ttg atc 251
Leu His His Thr Phe His Glu Phe Ser Leu Lys Tyr Gly Pro Leu Ile
50                  55                  60                  65

cag ctc aag ctc ggc tcg atc ccg tgc gtc gtg gcc tcg acg ccc gag 299
Gln Leu Lys Leu Gly Ser Ile Pro Cys Val Val Ala Ser Thr Pro Glu
                70                  75                  80

ctc gcg aga gag ttt ctt aag acg aac gag ctc gcg ttc tcc tct cgc 347
Leu Ala Arg Glu Phe Leu Lys Thr Asn Glu Leu Ala Phe Ser Ser Arg
            85                  90                  95

aag cac tct acg gcc ata gac atc gtc acc tac gac tcg tcc ttt gct 395
Lys His Ser Thr Ala Ile Asp Ile Val Thr Tyr Asp Ser Ser Phe Ala
        100                 105                 110

ttc tct ccg tac gga ccc tac tgg aag tac atc aag aaa ctg tgt acc 443
Phe Ser Pro Tyr Gly Pro Tyr Trp Lys Tyr Ile Lys Lys Leu Cys Thr
    115                 120                 125

tac gag ctg ctc gga gcg agg aac ctc gga cac ttt cag ccc att agg 491
Tyr Glu Leu Leu Gly Ala Arg Asn Leu Gly His Phe Gln Pro Ile Arg
130                 135                 140                 145

aat ctc gag gtc agg tcc ttt ctg cag ctt ctg atg cac aag agc ttt 539
Asn Leu Glu Val Arg Ser Phe Leu Gln Leu Leu Met His Lys Ser Phe
                150                 155                 160

aag ggc gag agt gtg aat gtg aca gac gag ctg gtg agg ctg acg agc 587
Lys Gly Glu Ser Val Asn Val Thr Asp Glu Leu Val Arg Leu Thr Ser
            165                 170                 175

aat gtg ata tcc cac atg atg ctg agc ata agg tgc tcg gaa gat gaa 635
Asn Val Ile Ser His Met Met Leu Ser Ile Arg Cys Ser Glu Asp Glu
 
180                 185                 190
ggc gat gct gag gcg gcg aga aca gtg ata cgc gag gtg acg cag ata     683
Gly Asp Ala Glu Ala Ala Arg Thr Val Ile Arg Glu Val Thr Gln Ile
    195                 200                 205
ttt ggg gaa ttc gat gtt acg gac ata ata tgg ttt tgc aag aaa ttc     731
Phe Gly Glu Phe Asp Val Thr Asp Ile Ile Trp Phe Cys Lys Lys Phe
210                 215                 220                 225
gat ctg cag ggg ata aag aag agg tca gag gat att cag agg agg tat     779
Asp Leu Gln Gly Ile Lys Lys Arg Ser Glu Asp Ile Gln Arg Arg Tyr
                230                 235                 240
gat gct ttg ctc gag aag att att agt gat aga gag aga tcg agg agg     827
Asp Ala Leu Leu Glu Lys Ile Ile Ser Asp Arg Glu Arg Ser Arg Arg
            245                 250                 255
caa aat cgt gat aag cat ggt ggc ggt aac aat gag gag gcc aag gat     875
Gln Asn Arg Asp Lys His Gly Gly Gly Asn Asn Glu Glu Ala Lys Asp
        260                 265                 270
ttt ctt gat atg ttg ctt gat gtg atg gag agt ggg gac acg gag gtc     923
Phe Leu Asp Met Leu Leu Asp Val Met Glu Ser Gly Asp Thr Glu Val
    275                 280                 285
aaa ttc act aga gag cat ctc aag gct ttg att ctg gat ttc ttc acg     971
Lys Phe Thr Arg Glu His Leu Lys Ala Leu Ile Leu Asp Phe Phe Thr
290                 295                 300                 305
gcc ggt acg gac aca aca gcc ata gcc acc gag tgg gcc atc gcc gag     1019
Ala Gly Thr Asp Thr Thr Ala Ile Ala Thr Glu Trp Ala Ile Ala Glu
                310                 315                 320
ctc atc aac aac ccg aac gtc ttg aag aag gcc caa gaa gaa ata tcc     1067
Leu Ile Asn Asn Pro Asn Val Leu Lys Lys Ala Gln Glu Glu Ile Ser
            325                 330                 335
cgg atc atc gga acc aag cgg atc gta caa gaa tcc gac gcc cca gac     1115
Arg Ile Ile Gly Thr Lys Arg Ile Val Gln Glu Ser Asp Ala Pro Asp
        340                 345                 350
cta ccc tac ctc cag gcc atc atc aag gag acg ttc cgg ctc cac cca     1163
Leu Pro Tyr Leu Gln Ala Ile Ile Lys Glu Thr Phe Arg Leu His Pro
    355                 360                 365
ccg atc ccg atg ctc tcg cgt aag tcc acc tcc gat tgc acg gtc aac     1211
Pro Ile Pro Met Leu Ser Arg Lys Ser Thr Ser Asp Cys Thr Val Asn
370                 375                 380                 385
ggc tac aaa atc caa gcc aag agc ctc ttg ttc gtg aac ata tgg tcc     1259
Gly Tyr Lys Ile Gln Ala Lys Ser Leu Leu Phe Val Asn Ile Trp Ser
                390                 395                 400
atc ggt cga aac cct aat tac tgg gaa agc cct atg gag ttc agg ccc     1307
Ile Gly Arg Asn Pro Asn Tyr Trp Glu Ser Pro Met Glu Phe Arg Pro
            405                 410                 415
gag cgg ttc ttg gag aag gga cgc gag tcc atc gac gtc aag ggc cag     1355
Glu Arg Phe Leu Glu Lys Gly Arg Glu Ser Ile Asp Val Lys Gly Gln
        420                 425                 430
cac ttt gag ctc ttg cct ttt ggg acg ggc cgc agg ggc tgt ccc ggt     1403
His Phe Glu Leu Leu Pro Phe Gly Thr Gly Arg Arg Gly Cys Pro Gly
    435                 440                 445
atg ttg ctg gct ata caa gag gtg gtc agc atc att ggg acc atg gtt     1451
Met Leu Leu Ala Ile Gln Glu Val Val Ser Ile Ile Gly Thr Met Val
450                 455                 460                 465
cag tgc ttc gac tgg aaa ttg gca gat ggt tcg ggc aat aat gtg gac     1499
Gln Cys Phe Asp Trp Lys Leu Ala Asp Gly Ser Gly Asn Asn Val Asp
                470                 475                 480
atg acc gaa cgg tct gga ttg acc gct ccg aga gcg ttc gat ctg gtt     1547
Met Thr Glu Arg Ser Gly Leu Thr Ala Pro Arg Ala Phe Asp Leu Val
            485                 490                 495
tgc cgg ttg tat cca cgg gtt gac ccg gcc aca ata tcg ggt gct         1592
Cys Arg Leu Tyr Pro Arg Val Asp Pro Ala Thr Ile Ser Gly Ala
 
        500                 505                 510
tgatgtagta gggtgaggcg cgtgttggtg tttcatcttt cggttttgtt ctgttagtat 1652
tattatggtc tgtgttgaag cctcaaggat ttt 1685
 <210> 4
   <211> 512
   <212> PRT
   <213> Torenia hybrida
   <220>
   <221>
   <222>
   <223> Amino acid sequence of flavone synthase
   <400> 4 
Met Asp Thr Val Leu Ile Thr Leu Tyr Thr Ala Leu Phe Val Ile Thr
1               5                   10                  15
Thr Thr Phe Leu Leu Leu Leu Arg Arg Arg Gly Pro Pro Ser Pro Pro
            20                  25                  30
Gly Pro Leu Ser Leu Pro Ile Ile Gly His Leu His Leu Leu Gly Pro
        35                  40                  45
Arg Leu His His Thr Phe His Glu Phe Ser Leu Lys Tyr Gly Pro Leu
    50                  55                  60
Ile Gln Leu Lys Leu Gly Ser Ile Pro Cys Val Val Ala Ser Thr Pro
65                  70                  75                  80
Glu Leu Ala Arg Glu Phe Leu Lys Thr Asn Glu Leu Ala Phe Ser Ser
                85                  90                  95
Arg Lys His Ser Thr Ala Ile Asp Ile Val Thr Tyr Asp Ser Ser Phe
            100                 105                 110
Ala Phe Ser Pro Tyr Gly Pro Tyr Trp Lys Tyr Ile Lys Lys Leu Cys
        115                 120                 125
Thr Tyr Glu Leu Leu Gly Ala Arg Asn Leu Gly His Phe Gln Pro Ile
    130                 135                 140
Arg Asn Leu Glu Val Arg Ser Phe Leu Gln Leu Leu Met His Lys Ser
145                 150                 155                 160
Phe Lys Gly Glu Ser Val Asn Val Thr Asp Glu Leu Val Arg Leu Thr
                165                 170                 175
Ser Asn Val Ile Ser His Met Met Leu Ser Ile Arg Cys Ser Glu Asp
            180                 185                 190
Glu Gly Asp Ala Glu Ala Ala Arg Thr Val Ile Arg Glu Val Thr Gln
        195                 200                 205
Ile Phe Gly Glu Phe Asp Val Thr Asp Ile Ile Trp Phe Cys Lys Lys
    210                 215                 220
Phe Asp Leu Gln Gly Ile Lys Lys Arg Ser Glu Asp Ile Gln Arg Arg
225                 230                 235                 240
Tyr Asp Ala Leu Leu Glu Lys Ile Ile Ser Asp Arg Glu Arg Ser Arg
                245                 250                 255
Arg Gln Asn Arg Asp Lys His Gly Gly Gly Asn Asn Glu Glu Ala Lys
            260                 265                 270
Asp Phe Leu Asp Met Leu Leu Asp Val Met Glu Ser Gly Asp Thr Glu
        275                 280                 285
Val Lys Phe Thr Arg Glu His Leu Lys Ala Leu Ile Leu Asp Phe Phe
    290                 295                 300
Thr Ala Gly Thr Asp Thr Thr Ala Ile Ala Thr Glu Trp Ala Ile Ala
305                 310                 315                 320
Glu Leu Ile Asn Asn Pro Asn Val Leu Lys Lys Ala Gln Glu Glu Ile
                325                 330                 335
Ser Arg Ile Ile Gly Thr Lys Arg Ile Val Gln Glu Ser Asp Ala Pro
            340                 345                 350
Asp Leu Pro Tyr Leu Gln Ala Ile Ile Lys Glu Thr Phe Arg Leu His
        355                 360                 365
Pro Pro Ile Pro Met Leu Ser Arg Lys Ser Thr Ser Asp Cys Thr Val
    370                 375                 380
 
Asn Gly Tyr Lys Ile Gln Ala Lys Ser Leu Leu Phe Val Asn Ile Trp
385                 390                 395                 400
Ser Ile Gly Arg Asn Pro Asn Tyr Trp Glu Ser Pro Met Glu Phe Arg
                405                 410                 415
Pro Glu Arg Phe Leu Glu Lys Gly Arg Glu Ser Ile Asp Val Lys Gly
            420                 425                 430
Gln His Phe Glu Leu Leu Pro Phe Gly Thr Gly Arg Arg Gly Cys Pro
        435                 440                 445
Gly Met Leu Leu Ala Ile Gln Glu Val Val Ser Ile Ile Gly Thr Met
    450                 455                 460
Val Gln Cys Phe Asp Trp Lys Leu Ala Asp Gly Ser Gly Asn Asn Val
465                 470                 475                 480
Asp Met Thr Glu Arg Ser Gly Leu Thr Ala Pro Arg Ala Phe Asp Leu
                485                 490                 495
Val Cys Arg Leu Tyr Pro Arg Val Asp Pro Ala Thr Ile Ser Gly Ala
            500                 505                 510
 <210> 5
   <211> 1770
   <212> DNA
   <213> Perilla frutescens
   <22C>
   <221> CDS
   <222> (21)...(1538)
   <223> Nucleotide sequence encoding flavone synthase
   <400> 5 
tgtcgacgga gcaagtggaa atg gca ctg tac gcc gcc ctc ttc ctc ctg 50
Met Ala Leu Tyr Ala Ala Leu Phe Leu Leu
1               5                   10
tcc gcc gcc gtg gtc cgc tcc gtt ctg gat cga aaa cgc ggg cgg ccg 98
Ser Ala Ala Val Val Arg Ser Val Leu Asp Arg Lys Arg Gly Arg Pro
                15                  20                  25
ccc tac cct ccc ggg ccg ttc cct ctt ccc atc atc ggc cac tta cac 146
Pro Tyr Pro Pro Gly Pro Phe Pro Leu Pro Ile Ile Gly His Leu His
            30                  35                  40
ctc ctc ggg ccg aga ctc cac caa acc ttc cac gat ctg tcc caa cgg 194
Leu Leu Gly Pro Arg Leu His Gln Thr Phe His Asp Leu Ser Gln Arg
        45                  50                  55
tac ggg ccc tta atg cag ctc cgc ctc ggg tcc atc cgc tgc gtc att 242
Tyr Gly Pro Leu Met Gln Leu Arg Leu Gly Ser Ile Arg Cys Val Ile
    60                  65                  70
gct gcc tcg ccg gag ctc gcc aag gaa tgc ctc aag aca cac gag ctc 290
Ala Ala Ser Pro Glu Leu Ala Lys Glu Cys Leu Lys Thr His Glu Leu
75                  80                  85                  90
gtc ttc tcc tcc cgc aaa cac tcc acc gcc att gat atc gtc acc tac 338
Val Phe Ser Ser Arg Lys His Ser Thr Ala Ile Asp Ile Val Thr Tyr
                95                  100                 105
gat tca tcc ttc gct ttc tct ccc tac ggg cct tac tgg aaa ttc atc 386
Asp Ser Ser Phe Ala Phe Ser Pro Tyr Gly Pro Tyr Trp Lys Phe Ile
            110                 115                 120
aag aaa tta tgc acc tac gag ctg ctc ggg gcc cga aat ctc gcc cac 434
Lys Lys Leu Cys Thr Tyr Glu Leu Leu Gly Ala Arg Asn Leu Ala His
        125                 130                 135
ttt cag ccc atc agg act ctc gaa gtc aag tct ttc ctc caa att ctt 482
Phe Gln Pro Ile Arg Thr Leu Glu Val Lys Ser Phe Leu Gln Ile Leu
    140                 145                 150
atg cgc aag ggt gaa tcg ggg gag agc ttc aac gtg act gag gag ctc 530
Met Arg Lys Gly Glu Ser Gly Glu Ser Phe Asn Val Thr Glu Glu Leu
155                 160                 165                 170
gtg aag ctg acg agc aac gtc ata tcg cat atg atg ctg agc ata cgg 578
Val Lys Leu Thr Ser Asn Val Ile Ser His Met Met Leu Ser Ile Arg
 
                175                 180                 185
tgt tca gag acg gag tcg gag gcg gag gcg gcg agg acg gtg att cgg 626
Cys Ser Glu Thr Glu Ser Glu Ala Glu Ala Ala Arg Thr Val Ile Arg
            190                 195                 200
gag gtc acg cag ata ttt ggg gag ttc gac gtc tcc gac atc ata tgg 674
Glu Val Thr Gln Ile Phe Gly Glu Phe Asp Val Ser Asp Ile Ile Trp
        205                 210                 215
ctt tgt aag aac ttc gat ttc caa ggt ata agg aag cgg tcc gag gat 722
Leu Cys Lys Asn Phe Asp Phe Gln Gly Ile Arg Lys Arg Ser Glu Asp
    220                 225                 230
atc cag agg aga tat gat gct ctg ctg gag aag atc atc acc gac aga 770
Ile Gln Arg Arg Tyr Asp Ala Leu Leu Glu Lys Ile Ile Thr Asp Arg
235                 240                 245                 250
gag aag cag agg cgg acc cac ggc ggc ggt ggc ggc ggc ggg gaa gcc 818
Glu Lys Gln Arg Arg Thr His Gly Gly Gly Gly Gly Gly Gly Glu Ala
                255                 260                 265
aag gat ttt ctt gac atg ttc ctc gac ata atg gag agc ggg aaa gcc 866
Lys Asp Phe Leu Asp Met Phe Leu Asp Ile Met Glu Ser Gly Lys Ala
            270                 275                 280
gaa gtt aaa ttc acg agg gag cat ctc aaa gct ttg att ctg gat ttc 914
Glu Val Lys Phe Thr Arg Glu His Leu Lys Ala Leu Ile Leu Asp Phe
        285                 290                 295
ttc acc gcc ggc acc gac acg acg gcg atc gtg tgt gaa tgg gcg ata 962
Phe Thr Ala Gly Thr Asp Thr Thr Ala Ile Val Cys Glu Trp Ala Ile
    300                 305                 310
gca gaa gtg atc aac aat cca aat gtg ttg aag aaa gct caa gaa gag 1010
Ala Glu Val Ile Asn Asn Pro Asn Val Leu Lys Lys Ala Gln Glu Glu
315                 320                 325                 330
att gcc aac atc gtc gga ttc gac aga att ctg caa gaa tcc gac gcc 1058
Ile Ala Asn Ile Val Gly Phe Asp Arg Ile Leu Gln Glu Ser Asp Ala
                335                 340                 345
cca aat ctg ccc tac ctt caa gcc ctc atc aaa gaa aca ttc cgg ctc 1106
Pro Asn Leu Pro Tyr Leu Gln Ala Leu Ile Lys Glu Thr Phe Arg Leu
            350                 355                 360
cac cct cca atc cca atg ctg gcg agg aaa tcg atc tcc gac tgc gtc 1154
His Pro Pro Ile Pro Met Leu Ala Arg Lys Ser Ile Ser Asp Cys Val
        365                 370                 375
atc gac ggc tac atg att ccg gcc aac acg ctg ctc ttc gtc aac ctc 1202
Ile Asp Gly Tyr Met Ile Pro Ala Asn Thr Leu Leu Phe Val Asn Leu
    380                 385                 390
tgg tcc atg ggg cgg aac cct aaa atc tgg gac tac ccg acg gcg ttc 1250
Trp Ser Met Gly Arg Asn Pro Lys Ile Trp Asp Tyr Pro Thr Ala Phe
395                 400                 405                 410
cag ccg gag agg ttt ctg gag aag gaa aag gcc gcc atc gat gtt aaa 1298
Gln Pro Glu Arg Phe Leu Glu Lys Glu Lys Ala Ala Ile Asp Val Lys
                415                 420                 425
ggg cag cat ttt gag ctg cta ccg ttc gga acg ggc agg aga ggc tgc 1346
Gly Gln His Phe Glu Leu Leu Pro Phe Gly Thr Gly Arg Arg Gly Cys
            430                 435                 440
cca ggg atg ctt tta gcc att cag gag gtg gtc atc ata att ggg acg 1394
Pro Gly Met Leu Leu Ala Ile Gln Glu Val Val Ile Ile Ile Gly Thr
        445                 450                 455
atg att caa tgc ttc gat tgg aag ctg ccc gac ggc tcc ggc cat gtt 1442
Met Ile Gln Cys Phe Asp Trp Lys Leu Pro Asp Gly Ser Gly His Val
    460                 465                 470
gat atg gca gaa cgg cca ggg ctc acg gca ccg cga gag acc gat ttg 1490
Asp Met Ala Glu Arg Pro Gly Leu Thr Ala Pro Arg Glu Thr Asp Leu
475                 480                 485                 490
ttt tgc cgt gtg gtg ccg cga gtt gat ccg ttg gtt gtt tcc acc cag 1538
Phe Cys Arg Val Val Pro Arg Val Asp Pro Leu Val Val Ser Thr Gln
 
                495                 500                 505
tgatcacccc ctttaaattt attaatgata tatttttatt ttgagaaaaa ataaaaatgc 1598
taattgtttt gtttcatgat gtaattgtta attagtttct attgtgcgct gtcgcgtgtc 1658
gcgtggctta agataagatt gtatcattgg tacctaggat gtattttcat tttcaataaa 1718
ttattttgtg ctgtgtatat taaaaaaaaa aaagaaaaaa aaaaaaaaaa aa 1770
 <210> 6
   <211> 506
   <212> PRT
   <213> Perilla frutescens
   <220>
   <221>
   <222>
   <223> Amino acid sequence of flavone synthase
   <400> 6 
Met Ala Leu Tyr Ala Ala Leu Phe Leu Leu Ser Ala Ala Val Val Arg
1               5                   10                  15
Ser Val Leu Asp Arg Lys Arg Gly Arg Pro Pro Tyr Pro Pro Gly Pro
            20                  25                  30
Phe Pro Leu Pro Ile Ile Gly His Leu His Leu Leu Gly Pro Arg Leu
        35                  40                  45
His Gln Thr Phe His Asp Leu Ser Gln Arg Tyr Gly Pro Leu Met Gln
    50                  55                  60
Leu Arg Leu Gly Ser Ile Arg Cys Val Ile Ala Ala Ser Pro Glu Leu
65                  70                  75                  80
Ala Lys Glu Cys Leu Lys Thr His Glu Leu Val Phe Ser Ser Arg Lys
                85                  90                  95
His Ser Thr Ala Ile Asp Ile Val Thr Tyr Asp Ser Ser Phe Ala Phe
            100                 105                 110
Ser Pro Tyr Gly Pro Tyr Trp Lys Phe Ile Lys Lys Leu Cys Thr Tyr
        115                 120                 125
Glu Leu Leu Gly Ala Arg Asn Leu Ala His Phe Gln Pro Ile Arg Thr
    130                 135                 140
Leu Glu Val Lys Ser Phe Leu Gln Ile Leu Met Arg Lys Gly Glu Ser
145                 150                 155                 160
Gly Glu Ser Phe Asn Val Thr Glu Glu Leu Val Lys Leu Thr Ser Asn
                165                 170                 175
Val Ile Ser His Met Met Leu Ser Ile Arg Cys Ser Glu Thr Glu Ser
            180                 185                 190
Glu Ala Glu Ala Ala Arg Thr Val Ile Arg Glu Val Thr Gln Ile Phe
        195                 200                 205
Gly Glu Phe Asp Val Ser Asp Ile Ile Trp Leu Cys Lys Asn Phe Asp
    210                 215                 220
Phe Gln Gly Ile Arg Lys Arg Ser Glu Asp Ile Gln Arg Arg Tyr Asp
225                 230                 235                 240
Ala Leu Leu Glu Lys Ile Ile Thr Asp Arg Glu Lys Gln Arg Arg Thr
                245                 250                 255
His Gly Gly Gly Gly Gly Gly Gly Glu Ala Lys Asp Phe Leu Asp Met
            260                 265                 270
Phe Leu Asp Ile Met Glu Ser Gly Lys Ala Glu Val Lys Phe Thr Arg
        275                 280                 285
Glu His Leu Lys Ala Leu Ile Leu Asp Phe Phe Thr Ala Gly Thr Asp
    290                 295                 300
Thr Thr Ala Ile Val Cys Glu Trp Ala Ile Ala Glu Val Ile Asn Asn
305                 310                 315                 320
Pro Asn Val Leu Lys Lys Ala Gln Glu Glu Ile Ala Asn Ile Val Gly
                325                 330                 335
Phe Asp Arg Ile Leu Gln Glu Ser Asp Ala Pro Asn Leu Pro Tyr Leu
            340                 345                 350
Gln Ala Leu Ile Lys Glu Thr Phe Arg Leu His Pro Pro Ile Pro Met
        355                 360                 365
 
Leu Ala Arg Lys Ser Ile Ser Asp Cys Val Ile Asp Gly Tyr Met Ile
    370                 375                 380
Pro Ala Asn Thr Leu Leu Phe Val Asn Leu Trp Ser Met Gly Arg Asn
385                 390                 395                 400
Pro Lys Ile Trp Asp Tyr Pro Thr Ala Phe Gln Pro Glu Arg Phe Leu
                405                 410                 415
Glu Lys Glu Lys Ala Ala Ile Asp Val Lys Gly Gln His Phe Glu Leu
            420                 425                 430
Leu Pro Phe Gly Thr Gly Arg Arg Gly Cys Pro Gly Met Leu Leu Ala
        435                 440                 445
Ile Gln Glu Val Val Ile Ile Ile Gly Thr Met Ile Gln Cys Phe Asp
    450                 455                 460
Trp Lys Leu Pro Asp Gly Ser Gly His Val Asp Met Ala Glu Arg Pro
465                 470                 475                 480
Gly Leu Thr Ala Pro Arg Glu Thr Asp Leu Phe Cys Arg Val Val Pro
                485                 490                 495
Arg Val Asp Pro Leu Val Val Ser Thr Gln
            500                 505
 <210> 7
   <211> 1781
   <212> DNA
   <213> Viola x wittrockiana
   <220>
   <221> CDS
   <222> (7)...(1524)
   <223> Nucleotide sequence encoding flavonoid-3',5'-hydroxylase
   <400> 7 
   gacaac atg gca att cta gtc acc gac ttc gtt gtc gcg gct ata att 48
   Met Ala Ile Leu Val Thr Asp Phe Val Val Ala Ala Ile Ile
   1 5 10
   ttc ttg atc act cgg ttc tta gtt cgt tct ctt ttc aag aaa cca acc 96
   Phe Leu Ile Thr Arg Phe Leu Val Arg Ser Leu Phe Lys Lys Pro Thr
   15 20 25 30
   cga ccg ctc ccc ccg ggt cct ctc ggt tgg ccc ttg gtg ggc gcc ctc 144
   Arg Pro Leu Pro Pro Gly Pro Leu Gly Trp Pro Leu Val Gly Ala Leu
   35 40 45
   cct ctc cta ggc gcc atg cct cac gtc gca cta gcc aaa ctc gct aag 192
   Pro Leu Leu Gly Ala Met Pro His Val Ala Leu Ala Lys Leu Ala Lys
   50 55 60
   aag tat ggt ccg atc atg cac cta aaa atg ggc acg tgc gac atg gtg 240
   Lys Tyr Gly Pro Ile Met His Leu Lys Met Gly Thr Cys Asp Met Val
   65 70 75
   gtc gcg tcc acc ccc gag tcg gct cga gcc ttc ctc aaa acg cta gac 288
   Val Ala Ser Thr Pro Glu Ser Ala Arg Ala Phe Leu Lys Thr Leu Asp
   80 85 90
   ctc aac ttc tcc aac cgc cca ccc aac gcg ggc gca tcc cac cta gcg 336
   Leu Asn Phe Ser Asn Arg Pro Pro Asn Ala Gly Ala Ser His Leu Ala
   95 100 105 110
   tac ggc gcg cag gac tta gtc ttc gcc aag tac ggt ccg agg tgg aag 384
   Tyr Gly Ala Gln Asp Leu Val Phe Ala Lys Tyr Gly Pro Arg Trp Lys
   115 120 125
   act tta aga aaa ttg agc aac ctc cac atg cta ggc ggg aag gcg ttg 432
   Thr Leu Arg Lys Leu Ser Asn Leu His Met Leu Gly Gly Lys Ala Leu
   130 135 140
   gat gat tgg gca aat gtg agg gtc acc gag cta ggc cac atg ctt aaa 480
   Asp Asp Trp Ala Asn Val Arg Val Thr Glu Leu Gly His Met Leu Lys
   145 150 155
   gcc atg tgc gag gcg agc cgg tgc ggg gag ccc gtg gtg ctg gcc gag 528
   Ala Met Cys Glu Ala Ser Arg Cys Gly Glu Pro Val Val Leu Ala Glu
   160 165 170
   atg ctc acg tac gcc atg gcg aac atg atc ggt caa gtg ata ctc agc 576
   Met Leu Thr Tyr Ala Met Ala Asn Met Ile Gly Gln Val Ile Leu Ser
   175 180 185 190
   cgg cgc gtg ttc gtg acc aaa ggg acc gag tct aac gag ttc aaa gac 624
   Arg Arg Val Phe Val Thr Lys Gly Thr Glu Ser Asn Glu Phe Lys Asp
   195 200 205
   atg gtg gtc gag ttg atg acg tcc gcc ggg tac ttc aac atc ggt gac 672
   Met Val Val Glu Leu Met Thr Ser Ala Gly Tyr Phe Asn Ile Gly Asp
   210 215 220
   ttc ata ccc tcg atc gct tgg atg gat ttg caa ggg atc gag cga ggg 720
   Phe Ile Pro Ser Ile Ala Trp Met Asp Leu Gln Gly Ile Glu Arg Gly
   225 230 235
   atg aag aag ctg cac acg aag ttt gat gtg tta ttg acg aag atg gtg 768
   Met Lys Lys Leu His Thr Lys Phe Asp Val Leu Leu Thr Lys Met Val
   240 245 250
   aag gag cat aga gcg acg agt cat gag cgc aaa ggg aag gca gat ttc 816
   Lys Glu His Arg Ala Thr Ser His Glu Arg Lys Gly Lys Ala Asp Phe
   255 260 265 270
   ctc gac gtt ctc ttg gaa gaa tgc gac aat aca aat ggg gag aag ctt 864
   Leu Asp Val Leu Leu Glu Glu Cys Asp Asn Thr Asn Gly Glu Lys Leu
   275 280 285
   agt att acc aat atc aaa gct gtc ctt ttg aat cta ttc acg gcg ggc 912
   Ser Ile Thr Asn Ile Lys Ala Val Leu Leu Asn Leu Phe Thr Ala Gly
   290 295 300
   acg gac aca tct tcg agc ata atc gaa tgg gcg tta acg gag atg atc 960
   Thr Asp Thr Ser Ser Ser Ile Ile Glu Trp Ala Leu Thr Glu Met Ile
   305 310 315
   aag aat ccg acg atc tta aaa aag gcg caa gag gag atg gat cga gtc 1008
   Lys Asn Pro Thr Ile Leu Lys Lys Ala Gln Glu Glu Met Asp Arg Val
   320 325 330
   atc ggt cgt gat cgg agg ctg ctc gaa tcg gac ata tcg agc ctc ccg 1056
   Ile Gly Arg Asp Arg Arg Leu Leu Glu Ser Asp Ile Ser Ser Leu Pro
   335 340 345 350
   tac cta caa gcc att gct aaa gaa acg tat cgc aaa cac ccg tcg acg 1104
   Tyr Leu Gln Ala Ile Ala Lys Glu Thr Tyr Arg Lys His Pro Ser Thr
   355 360 365
   cct ctc aac ttg ccg agg att gcg atc caa gca tgt gaa gtt gat ggc 1152
   Pro Leu Asn Leu Pro Arg Ile Ala Ile Gln Ala Cys Glu Val Asp Gly
   370 375 380
   tac tac atc cct aag gac gcg agg ctt agc gtg aac att tgg gcg atc 1200
   Tyr Tyr Ile Pro Lys Asp Ala Arg Leu Ser Val Asn Ile Trp Ala Ile
   385 390 395
   ggt cgg gac ccg aat gtt tgg gag aat ccg ttg gag ttc ttg ccg gaa 1248
   Gly Arg Asp Pro Asn Val Trp Glu Asn Pro Leu Glu Phe Leu Pro Glu
   400 405 410
   aga ttc ttg tct gaa gag aat ggg aag atc aat ccc ggt ggg aat gat 1296
   Arg Phe Leu Ser Glu Glu Asn Gly Lys Ile Asn Pro Gly Gly Asn Asp
   415 420 425 430
   ttt gag ctg att ccg ttt gga gcc ggg agg aga att tgt gcg ggg aca 1344
   Phe Glu Leu Ile Pro Phe Gly Ala Gly Arg Arg Ile Cys Ala Gly Thr
   435 440 445
   agg atg gga atg gtc ctt gta agt tat att ttg ggc act ttg gtc cat 1392
   Arg Met Gly Met Val Leu Val Ser Tyr Ile Leu Gly Thr Leu Val His
   450 455 460
   tct ttt gat tgg aaa tta cca aat ggt gtc gct gag ctt aat atg gat 1440
   Ser Phe Asp Trp Lys Leu Pro Asn Gly Val Ala Glu Leu Asn Met Asp
   465 470 475
   gaa agt ttt ggg ctt gca ttg caa aag gcc gtg ccg ctc tcg gcc ttg 1488
   Glu Ser Phe Gly Leu Ala Leu Gln Lys Ala Val Pro Leu Ser Ala Leu
   480 485 490
   gtc agc cca cgg ttg gcc tca aac gcg tac gca acc tgagctaatg 1534
   Val Ser Pro Arg Leu Ala Ser Asn Ala Tyr Ala Thr
   495 500 505
   ggctgggcct agtttgtggg gccttaattt agagactttt gtgttttaag gtgtgtactt 1594
   tattaattgg gtgcttaaat gtgtgtttta atttgtattt atggttaatt atgactttat 1654
   tgtataatta tttatttttc ccttctgggt attttatcca tttaattttt cttcagaatt 1714
   atgatcatag ttatcagaat aaaattgaaa ataatgaatc ggaaaaaaaa aaaaaaaaaa 1774
   aaaaaaa 1781
 <210> 8
   <211> 506
   <212> PRT
   <213> Viola x wittrockiana
   <220>
   <221>
   <222>
   <223> Amino acid sequence of flavonoid-3',5'-hydroxylase
   <400> 8 
Met Ala Ile Leu Val Thr Asp Phe Val Val Ala Ala Ile Ile Phe Leu
1               5                   10                  15
Ile Thr Arg Phe Leu Val Arg Ser Leu Phe Lys Lys Pro Thr Arg Pro
            20                  25                  30
Leu Pro Pro Gly Pro Leu Gly Trp Pro Leu Val Gly Ala Leu Pro Leu
        35                  40                  45
Leu Gly Ala Met Pro His Val Ala Leu Ala Lys Leu Ala Lys Lys Tyr
    50                  55                  60
Gly Pro Ile Met His Leu Lys Met Gly Thr Cys Asp Met Val Val Ala
65                  70                  75                  80
Ser Thr Pro Glu Ser Ala Arg Ala Phe Leu Lys Thr Leu Asp Leu Asn
                85                  90                  95
Phe Ser Asn Arg Pro Pro Asn Ala Gly Ala Ser His Leu Ala Tyr Gly
            100                 105                 110
Ala Gln Asp Leu Val Phe Ala Lys Tyr Gly Pro Arg Trp Lys Thr Leu
        115                 120                 125
Arg Lys Leu Ser Asn Leu His Met Leu Gly Gly Lys Ala Leu Asp Asp
    130                 135                 140
Trp Ala Asn Val Arg Val Thr Glu Leu Gly His Met Leu Lys Ala Met
145                 150                 155                 160
Cys Glu Ala Ser Arg Cys Gly Glu Pro Val Val Leu Ala Glu Met Leu
                165                 170                 175
Thr Tyr Ala Met Ala Asn Met Ile Gly Gln Val Ile Leu Ser Arg Arg
            180                 185                 190
Val Phe Val Thr Lys Gly Thr Glu Ser Asn Glu Phe Lys Asp Met Val
        195                 200                 205
Val Glu Leu Met Thr Ser Ala Gly Tyr Phe Asn Ile Gly Asp Phe Ile
    210                 215                 220
Pro Ser Ile Ala Trp Met Asp Leu Gln Gly Ile Glu Arg Gly Met Lys
225                 230                 235                 240
Lys Leu His Thr Lys Phe Asp Val Leu Leu Thr Lys Met Val Lys Glu
                245                 250                 255
His Arg Ala Thr Ser His Glu Arg Lys Gly Lys Ala Asp Phe Leu Asp
            260                 265                 270
Val Leu Leu Glu Glu Cys Asp Asn Thr Asn Gly Glu Lys Leu Ser Ile
        275                 280                 285
Thr Asn Ile Lys Ala Val Leu Leu Asn Leu Phe Thr Ala Gly Thr Asp
    290                 295                 300
Thr Ser Ser Ser Ile Ile Glu Trp Ala Leu Thr Glu Met Ile Lys Asn
305                 310                 315                 320
Pro Thr Ile Leu Lys Lys Ala Gln Glu Glu Met Asp Arg Val Ile Gly
                325                 330                 335
Arg Asp Arg Arg Leu Leu Glu Ser Asp Ile Ser Ser Leu Pro Tyr Leu
 
340                 345                 350
Gln Ala Ile Ala Lys Glu Thr Tyr Arg Lys His Pro Ser Thr Pro Leu
        355                 360                 365
Asn Leu Pro Arg Ile Ala Ile Gln Ala Cys Glu Val Asp Gly Tyr Tyr
    370                 375                 380
Ile Pro Lys Asp Ala Arg Leu Ser Val Asn Ile Trp Ala Ile Gly Arg
385                 390                 395                 400
Asp Pro Asn Val Trp Glu Asn Pro Leu Glu Phe Leu Pro Glu Arg Phe
                405                 410                 415
Leu Ser Glu Glu Asn Gly Lys Ile Asn Pro Gly Gly Asn Asp Phe Glu
            420                 425                 430
Leu Ile Pro Phe Gly Ala Gly Arg Arg Ile Cys Ala Gly Thr Arg Met
        435                 440                 445
Gly Met Val Leu Val Ser Tyr Ile Leu Gly Thr Leu Val His Ser Phe
    450                 455                 460
Asp Trp Lys Leu Pro Asn Gly Val Ala Glu Leu Asn Met Asp Glu Ser
465                 470                 475                 480
Phe Gly Leu Ala Leu Gln Lys Ala Val Pro Leu Ser Ala Leu Val Ser
                485                 490                 495
Pro Arg Leu Ala Ser Asn Ala Tyr Ala Thr
            500                 505
 <210> 9
   <211> 1014
   <212> DNA
   <213> Torenia hybrida
   <220>
   <221> CDS
   <222> (69)...(785)
   <223> Nucleotide sequence encoding methyltransferase
   <400> 9 
cggcacgagt tcaattccgc cattttctcc aataataaca ttcataaata caatcagcag 60
cagcaaaa atg aaa gat aag ttc tat ggc acc att ttg cag agc gaa 107
Met Lys Asp Lys Phe Tyr Gly Thr Ile Leu Gln Ser Glu
1               5                   10
gcc ctc gca aag tat ctg tta gag aca agt gcc tat cca cga gaa cat 155
Ala Leu Ala Lys Tyr Leu Leu Glu Thr Ser Ala Tyr Pro Arg Glu His
    15                  20                  25
ccg cag ctc aaa gaa cta agg agc gca act gtg gac aag tat caa tat 203
Pro Gln Leu Lys Glu Leu Arg Ser Ala Thr Val Asp Lys Tyr Gln Tyr
30                  35                  40                  45
tgg agc ttg atg aat gtt cca gct gat gag ggg cag ttc att tca atg 251
Trp Ser Leu Met Asn Val Pro Ala Asp Glu Gly Gln Phe Ile Ser Met
                50                  55                  60
tta ctg aaa att atg aac gca aaa aag aca att gaa gtt gga gtt ttc 299
Leu Leu Lys Ile Met Asn Ala Lys Lys Thr Ile Glu Val Gly Val Phe
            65                  70                  75
aca ggc tac tca ctc cta tca act gct ctg gct cta cct gat gat ggc 347
Thr Gly Tyr Ser Leu Leu Ser Thr Ala Leu Ala Leu Pro Asp Asp Gly
        80                  85                  90
aaa atc gtt gcc att gat cct gat aga gaa gct tat gag act ggt ttg 395
Lys Ile Val Ala Ile Asp Pro Asp Arg Glu Ala Tyr Glu Thr Gly Leu
    95                  100                 105
cca ttt atc aag aaa gca aac gtg gct cat aaa atc caa tac ata caa 443
Pro Phe Ile Lys Lys Ala Asn Val Ala His Lys Ile Gln Tyr Ile Gln
110                 115                 120                 125
tct gat gcc atg aaa gtc atg aat gac ctc att gct gcc aag gga gaa 491
Ser Asp Ala Met Lys Val Met Asn Asp Leu Ile Ala Ala Lys Gly Glu
                130                 135                 140
gaa gaa gag ggg agc ttt gac ttt ggg ttc gtg gat gca gac aaa gaa 539
Glu Glu Glu Gly Ser Phe Asp Phe Gly Phe Val Asp Ala Asp Lys Glu
 
            145                 150                 155
aac tac ata aac tac cac gag aaa ctg ttg aag ctg gtt aag gtt gga 587
Asn Tyr Ile Asn Tyr His Glu Lys Leu Leu Lys Leu Val Lys Val Gly
        160                 165                 170
ggg atc ata gga tac gac aac act ctg tgg tct gga aca gtt gct gca 635
Gly Ile Ile Gly Tyr Asp Asn Thr Leu Trp Ser Gly Thr Val Ala Ala
    175                 180                 185
tct gaa gac gat gag aat aat atg caa gac tac tta aga ggt tgc aga 683
Ser Glu Asp Asp Glu Asn Asn Met Gln Asp Tyr Leu Arg Gly Cys Arg
190                 195                 200                 205
ggg cat atc ctc aaa cta aac tcc ttt ctc gca aac gat gat cgg att 731
Gly His Ile Leu Lys Leu Asn Ser Phe Leu Ala Asn Asp Asp Arg Ile
                210                 215                 220
gaa ttg gct cac ctc tct att gga gat gga ctc acc ttg tgc aaa cgt 779
Glu Leu Ala His Leu Ser Ile Gly Asp Gly Leu Thr Leu Cys Lys Arg
            225                 230                 235
ctc aaa taataatttt caactttatt attattgttt cataaaaagc atttactgct 835
Leu Lys
ggcctggcct ggcctgtttc agcatcttat atttctattg ttctaaatat tttagttatc 895
ttgtttatca acttgtctgt cttatatgtt taaaagaaag atgtcatgta attgtaactc 955
gatcgggctc ttgtaatatt ataatgaatt ttattgattt caaaaaaaaa aaaaaaaaa 1014
 <210>10
   <211>239
   <212> PRT
   <213>Torenia hybrida
   <220>
   <221>
   <222>
   <223>Amino acid sequence of methyltransferase <400>10

Met Lys Asp Lys Phe Tyr Gly Thr Ile Leu Gln Ser Glu Ala Leu Ala
1               5                   10                  15
Lys Tyr Leu Leu Glu Thr Ser Ala Tyr Pro Arg Glu His Pro Gln Leu
            20                  25                  30
Lys Glu Leu Arg Ser Ala Thr Val Asp Lys Tyr Gln Tyr Trp Ser Leu
        35                  40                  45
Met Asn Val Pro Ala Asp Glu Gly Gln Phe Ile Ser Met Leu Leu Lys
    50                  55                  60
Ile Met Asn Ala Lys Lys Thr Ile Glu Val Gly Val Phe Thr Gly Tyr
65                  70                  75                  80
Ser Leu Leu Ser Thr Ala Leu Ala Leu Pro Asp Asp Gly Lys Ile Val
                85                  90                  95
Ala Ile Asp Pro Asp Arg Glu Ala Tyr Glu Thr Gly Leu Pro Phe Ile
            100                 105                 110
Lys Lys Ala Asn Val Ala His Lys Ile Gln Tyr Ile Gln Ser Asp Ala
        115                 120                 125
Met Lys Val Met Asn Asp Leu Ile Ala Ala Lys Gly Glu Glu Glu Glu
    130                 135                 140
Gly Ser Phe Asp Phe Gly Phe Val Asp Ala Asp Lys Glu Asn Tyr Ile
145                 150                 155                 160
Asn Tyr His Glu Lys Leu Leu Lys Leu Val Lys Val Gly Gly Ile Ile
                165                 170                 175
Gly Tyr Asp Asn Thr Leu Trp Ser Gly Thr Val Ala Ala Ser Glu Asp
            180                 185                 190
Asp Glu Asn Asn Met Gln Asp Tyr Leu Arg Gly Cys Arg Gly His Ile
        195                 200                 205
Leu Lys Leu Asn Ser Phe Leu Ala Asn Asp Asp Arg Ile Glu Leu Ala
    210                 215                 220
His Leu Ser Ile Gly Asp Gly Leu Thr Leu Cys Lys Arg Leu Lys
225                 230                 235

## Claims

1. A rose **characterized by** comprising a malvidin, a flavone and delphinidin added by a genetic modification method by expression of an anthocyanin methyltransferase gene, a flavone synthase gene and the pansy (*Viola x wittrockiana*) flavonoid 3',5'-hydroxylase gene.

2. A rose according to claim 1, wherein the flavone synthase gene is a flavone synthase gene derived from the family Scrophulariaceae.

3. A rose according to claim 2, wherein the flavone synthase gene derived from the family Scrophulariaceae is a flavone synthase gene derived from snapdragon of the family Scrophulariaceae (Scrophulariaceae, *Antirrhinum majus*).

4. A rose according to claim 2, wherein the flavone synthase gene derived from the family Scrophulariaceae is a flavone synthase gene derived from torenia of the family Scrophulariaceae (Scrophulariaceae, *Torenia hybrida*).

5. A rose according to claim 3, wherein the flavone synthase gene derived from snapdragon of the family Scrophulariaceae is a gene coding for
(1) flavone synthase having the amino acid sequence listed as SEQ ID NO: 2,
(2) flavone synthase having the amino acid sequence listed as SEQ ID NO: 2 modified by an addition or deletion of one or several amino acids and/or substitution of one or several amino acids by other amino acids,
(3) flavone synthase having an amino acid sequence with at least 90% sequence identity to the amino acid sequence listed as SEQ ID NO: 2, or
(4) flavone synthase encoded by nucleic acid that hybridizes with nucleic acid having the nucleotide sequence of SEQ ID NO: 1 under highly stringent conditions.

6. A rose according to claim 4, wherein the flavone synthase gene derived from torenia of the family Scrophulariaceae is a gene coding for
(1) flavone synthase having the amino acid sequence listed as SEQ ID NO: 4,
(2) flavone synthase having the amino acid sequence listed as SEQ ID NO: 4 modified by an addition or deletion of one or several amino acids and/or substitution of one or several amino acids by other amino acids,
(3) flavone synthase having an amino acid sequence with at least 90% sequence identity to the amino acid sequence listed as SEQ ID NO: 4, or
(4) flavone synthase encoded by nucleic acid that hybridizes with nucleic acid having the nucleotide sequence of SEQ ID NO: 3 under highly stringent conditions.

7. A rose according to any one of claims 1 to 6, wherein the pansy flavonoid 3',5'-hydroxylase gene is a gene coding for:
(1) flavonoid 3',5'-hydroxylase having the amino acid sequence listed as SEQ ID NO: 8,
(2) flavonoid 3',5'-hydroxylase having the amino acid sequence listed as SEQ ID NO: 8 modified by an addition or deletion of one or several amino acids and/or substitution of one or several amino acids by other amino acids,
(3) flavonoid 3',5'-hydroxylase having an amino acid sequence with at least 90% sequence identity to the amino acid sequence listed as SEQ ID NO: 8, or
(4) flavonoid 3',5'-hydroxylase encoded by nucleic acid that hybridizes with nucleic acid having the nucleotide sequence of SEQ ID NO: 7 under highly stringent conditions.

8. A rose according to any one of claims 1 to 7, wherein the anthocyanin methyltransferase gene is a gene coding for:
(1) methyltransferase having the amino acid sequence listed as SEQ ID NO: 10,
(2) methyltransferase having the amino acid sequence listed as SEQ ID NO: 10 modified by an addition or deletion of one or several amino acids and/or substitution of one or several amino acids by other amino acids,
(3) methyltransferase having an amino acid sequence with at least 90% sequence identity to the amino acid sequence listed as SEQ ID NO: 10, or
(4) methyltransferase encoded by nucleic acid that hybridizes with nucleic acid having the nucleotide sequence of SEQ ID NO: 9 under highly stringent conditions.

9. A rose according to any one of claims 1 to 8, wherein the flower color is changed with respect to the host.

10. A rose according to claim 9, wherein the change in flower color is a change toward blue.

11. A rose according to claim 9 or 10, wherein the change in flower color is a change such that the hue angle (θ) according to the L*a*b color system chromaticity diagram approaches 270° which is the blue axis.

12. A rose according to claim 9, wherein the change in flower color is a change such that the minimum value of the reflection spectrum of the petal shifts toward the longer wavelength end.

13. A rose portion, descendant, tissue, vegetative body or cell having the same properties as a rose according to any one of claims 1 to 12.

14. A method for modifying the flower color of a rose by a co-pigmentation effect produced by adding a flavone and malvidin by a genetic modification technique by expression of an anthocyanin methyltransferase gene, a flavone synthase gene and the pansy *(Viola x wittrockiana)* flavonoid 3',5'-hydroxylase gene.

15. The method according to claim 14, wherein the co-pigmentation effect is an effect of changing the flower color toward blue.

## Patentansprüche

1. Rose, **dadurch gekennzeichnet, dass** sie ein Malvidin, ein Flavon und Delphinidin umfasst, die durch ein genetisches Modifikationsverfahren durch Expression eines Anthocyan-Methyltransferasegens, eines Flavonsynthasegens und des Flavonoid-3',5'-Hydroxylasegens des Stiefmütterchens *(Viola x wittrockiana)* hinzugefügt wurden.

2. Rose gemäß Anspruch 1, wobei das Flavonsynthasegen ein Flavonsynthasegen ist, welches von der Familie Scrophulariaceae abgeleitet ist.

3. Rose gemäß Anspruch 2, wobei das von der Familie Scrophulariaceae abgeleitete Flavonsynthasegen ein Flavonsynthasegen ist, das vom Löwenmäulchen von der Familie Scrophulariaceae (Scrophulariaceae, *Antirrhinum majus*) abgeleitet ist.

4. Rose gemäß Anspruch 2, wobei das von der Familie Scrophulariaceae abgeleitete Flavonsynthasegen ein Flavonsynthasegen ist, das von der Torenia von der Familie Scrophulariaceae (Scrophulariaceae, *Torenia hybrida*) abgeleitet ist.

5. Rose gemäß Anspruch 3, wobei das vom Löwenmäulchen von der Familie Scrophulariaceae abgeleitete Flavonsynthasegen ein Gen ist, das codiert:
(1) eine Flavonsynthase, welche die Aminosäuresequenz wie in SEQ ID NO: 2 gezeigt hat,
(2) eine Flavonsynthase, welche die Aminosäuresequenz wie in SEQ ID NO: 2 gezeigt hat, die durch Addition oder Deletion einer oder mehrerer Aminosäuren und/oder Substitution einer oder mehrerer Aminosäuren durch andere Aminosäuren modifiziert ist,
(3) eine Flavonsynthase, welche eine Aminosäuresequenz mit einer mindestens 90%igen Sequenzidentität zu der in SEQ ID NO: 2 gezeigten Aminosäuresequenz hat, oder
(4) eine Flavonsynthase, welche durch eine Nucleinsäure codiert wird, die unter hoch stringenten Bedingungen mit der Nucleinsäure, die die Nucleotidsequenz von SEQ ID NO: 1 hat, hybridisiert.

6. Rose gemäß Anspruch 4, wobei das von der Torenia der Familie Scrophulariaceae abgeleitete Flavonsynthasegen ein Gen ist, das codiert:
(1) eine Flavonsynthase, welche die Aminosäuresequenz wie in SEQ ID NO: 4 gezeigt hat,
(2) eine Flavonsynthase, welche die Aminosäuresequenz wie in SEQ ID NO: 4 gezeigt hat, die durch Addition oder Deletion einer oder mehrerer Aminosäuren und/oder Substitution einer oder mehrerer Aminosäuren durch andere Aminosäuren modifiziert ist,
(3) eine Flavonsynthase, welche eine Aminosäuresequenz mit einer mindestens 90%igen Sequenzidentität zu der in SEQ ID NO: 4 gezeigten Aminosäuresequenz hat, oder
(4) eine Flavonsynthase, welche durch eine Nucleinsäure codiert wird, die unter hoch stringenten Bedingungen mit der Nucleinsäure, die die Nucleotidsequenz von SEQ ID NO: 3 hat, hybridisiert.

7. Rose gemäß einem der Ansprüche 1 bis 6, wobei das Stiefmütterchen-Flavonoid-3',5'-Hydroxylasegen ein Gen ist, das codiert:
(1) eine Flavonoid-3',5'-Hydroxylase, welche die Aminosäuresequenz wie in SEQ ID NO: 8 gezeigt hat,
(2) eine Flavonoid-3',5'-Hydroxylase, welche die Aminosäuresequenz wie in SEQ ID NO: 8 gezeigt hat, die durch Addition oder Deletion einer oder mehrerer Aminosäuren und/oder Substitution einer oder mehrerer Aminosäuren durch andere Aminosäuren modifiziert ist,
(3) eine Flavonoid-3',5'-Hydroxylase, welche eine Aminosäuresequenz mit einer mindestens 90%igen Sequenzidentität zu der in SEQ ID NO: 8 gezeigten Aminosäuresequenz hat, oder
(4) eine Flavonoid-3',5'-Hydroxylase, welche durch eine Nucleinsäure codiert wird, die unter hoch stringenten Bedingungen mit der Nucleinsäure, die die Nucleotidsequenz von SEQ ID NO: 7 hat, hybridisiert.

8. Rose gemäß einem der Ansprüche 1 bis 7, wobei das Anthocyan-Methyltransferasegen ein Gen ist, das codiert:
(1) eine Methyltransferase, welche die Aminosäuresequenz wie in SEQ ID NO: 10 gezeigt hat,
(2) eine Methyltransferase, welche die Aminosäuresequenz wie in SEQ ID NO: 10 gezeigt hat, die durch Addition oder Deletion einer oder mehrerer Aminosäuren und/oder Substitution einer oder mehrerer Aminosäuren durch andere Aminosäuren modifiziert ist,
(3) eine Methyltransferase, welche eine Aminosäuresequenz mit einer mindestens 90%igen Sequenzidentität zu der in SEQ ID NO: 10 gezeigten Aminosäuresequenz hat, oder
(4) eine Methyltransferase, welche durch eine Nucleinsäure codiert wird, die unter hoch stringenten Bedingungen mit der Nucleinsäure, die die Nucleotidsequenz von SEQ ID NO: 9 hat, hybridisiert.

9. Rose gemäß einem der Ansprüche 1 bis 8, wobei die Blütenfarbe in Bezug auf den Wirt geändert ist.

10. Rose gemäß Anspruch 9, wobei die Änderung der Blütenfarbe eine Änderung in Richtung blau ist.

11. Rose gemäß Anspruch 9 oder 10, wobei die Änderung der Blütenfarbe eine solche Änderung ist, dass der Farbenwinkel (θ) gemäß der L*a*b-Farbsystem-Farbtafel 270° erreicht, welches die blaue Achse ist.

12. Rose gemäß Anspruch 9, wobei die Änderung der Blütenfarbe eine solche Änderung ist, dass sich der Minimalwert des Reflektionspektrums des Blütenblatts in Richtung der längeren Wellenlänge verschiebt.

13. Rosenteil, Nachkomme, Gewebe, vegetativer Körper oder Zelle, welche dieselben Eigenschaften wie eine Rose gemäß einem der Ansprüche 1 bis 12 hat.

14. Verfahren zur Modifizierung der Blütenfarbe einer Rose durch einen Ko-Pigmentierungseffekt, der durch Hinzufügen eines Flavons und von Malvidin durch eine genetische Modifikationstechnik durch Expression eines Anthocyan-Methyltransferasegens, eines Flavonsynthasegens und dem Flavonoid-3',5'-Hydroxylasegen des Stiefmütterchens *(Viola x wittrockiana)* hergestellt wird.

15. Verfahren gemäß Anspruch 14, wobei der Ko-Pigmentierungseffekt ein Effekt ist, welcher die Blütenfarbe in Richtung blau ändert.

## Revendications

1. Une rose **caractérisée en ce qu'**elle comprend une malvidine, une flavone et une delphinidine ajoutées par une méthode de modification génétique par expression d'un gène de l'anthocyanine méthyltransférase, d'un gène de la flavone synthase et du gène de la flavonoïde 3',5'-hydroxylase de pensée (*Viola x wittrockiana*).

2. Une rose selon la revendication 1, dans laquelle le gène de la flavone synthase est un gène de la flavone synthase dérivée de la famille des Scrophulariaceae.

3. Une rose selon la revendication 2, dans laquelle le gène de la flavone synthase dérivée de la famille des Scrophulariaceae est un gène de la flavone synthase dérivée du muflier de la famille des Scrophulariaceae (Scrophulariaceae, *Antirrhinum majus*).

4. Une rose selon la revendication 2, dans laquelle le gène de la flavone synthase dérivée de la famille des Scrophulariaceae est un gène de la flavone synthase dérivée du torenia de la famille des Scrophulariaceae (Scrophulariaceae, *Torenia hybrida).*

5. Une rose selon la revendication 3, dans laquelle le gène de la flavone synthase dérivée de muflier de la famille des Scrophulariaceae est un gène codant pour
(1) une flavone synthase ayant la séquence d'acide aminé telle que définie par SEQ ID NO: 2,
(2) une flavone synthase ayant la séquence d'acide aminé telle que définie par SEQ ID NO: 2 modifiée par un ajout ou délétion d'un ou plusieurs acides aminés et/ou substitution d'un ou plusieurs acides aminés par d'autres acides aminés,
(3) une flavone synthase ayant une séquence d'acide aminé avec au moins 90% de séquence identique à la séquence d'acide aminé telle que définie par SEQ ID NO: 2, ou
(4) une flavone synthase codée par un acide nucléique qui, dans des conditions fortement stringentes, s'hybride avec l'acide nucléique ayant la séquence nucléotidique de SEQ ID NO: 1.

6. Une rose selon la revendication 4, dans laquelle le gène de la flavone synthase dérivée du torenia de la famille des Scrophulariaceae est un gène codant pour
(1) une flavone synthase ayant la séquence d'acide aminé telle que définie par SEQ ID NO: 4,
(2) une flavone synthase ayant la séquence d'acide aminé telle que définie par SEQ ID NO: 4 modifiée par un ajout ou délétion d'un ou plusieurs acides aminés et/ou substitution d'un ou plusieurs acides aminés par d'autres acides aminés,
(3) une flavone synthase ayant une séquence d'acide aminé avec au moins 90% de séquence identique à la séquence d'acide aminé telle que définie par SEQ ID NO: 4, ou
(4) une flavone synthase codée par un acide nucléique qui, dans des conditions fortement stringentes, s'hybride avec l'acide nucléique ayant la séquence nucléotidique de SEQ ID NO: 3.

7. Une rose selon l'une quelconque des revendications 1 à 6, dans laquelle le gène de la flavonoïde 3',5'-hydroxylase de pensée est un gène codant pour :
(1) une flavonoïde 3',5'-hydroxylase ayant la séquence d'acide aminé telle que définie par SEQ ID NO: 8,
(2) une flavonoïde 3',5'-hydroxylase ayant la séquence d'acide aminé telle que définie par SEQ ID NO: 8 modifiée par un ajout ou délétion d'un ou plusieurs acides aminés et/ou substitution d'un ou plusieurs acides aminés par d'autres acides aminés,
(3) une flavonoïde 3',5'-hydroxylase ayant une séquence d'acide aminé avec au moins 90% de séquence identique à la séquence d'acide aminé telle que définie par SEQ ID NO: 8, ou
(4) une flavonoïde 3',5'-hydroxylase codée par un acide nucléique qui, dans des conditions fortement stringentes, s'hybride avec l'acide nucléique ayant la séquence nucléotidique de SEQ ID NO: 7.

8. Une rose selon l'une quelconque des revendications 1 à 7, dans laquelle le gène de l'anthocyanine méthyltransférase est un gène codant pour :
(1) une méthyltransférase ayant la séquence d'acide aminé telle que définie par SEQ ID NO: 10,
(2) une méthyltransférase ayant la séquence d'acide aminé telle que définie par SEQ ID NO: 10 modifiée par un ajout ou délétion d'un ou plusieurs acides aminés et/ou substitution d'un ou plusieurs acides aminés par d'autres acides aminés,
(3) une méthyltransférase ayant une séquence d'acide aminé avec au moins 90% de séquence identique à la séquence d'acide aminé telle que définie par SEQ ID NO: 10, ou
(4) une méthyltransférase codée par un acide nucléique qui, dans des conditions fortement stringentes, s'hybride avec l'acide nucléique ayant la séquence nucléotidique de SEQ ID NO: 9.

9. Une rose selon l'une quelconque des revendications 1 à 8, dans laquelle la couleur de la fleur est modifiée par rapport à l'hôte.

10. Une rose selon la revendication 9, dans laquelle la modification de la couleur de la fleur est une modification vers le bleu.

11. Une rose selon la revendication 9 ou 10, dans laquelle la modification de la couleur de la fleur est une modification telle que l'angle de teinte (θ) selon le diagramme de chromaticité du système de couleur L*a*b avoisine 270° ce qui correspond à l'axe des bleus.

12. Une rose selon la revendication 9, dans laquelle la modification de la couleur de la fleur est une modification telle que la valeur minimum du spectre de réflexion du pétale se déplace vers l'extrémité de longueur d'onde la plus longue.

13. Une portion de rose, descendant, tissu, cellule ou corps végétatif ayant les mêmes propriétés qu'une rose selon l'une quelconque des revendications 1 à 12.

14. Un procédé pour modifier la couleur de la fleur d'une rose par un effet de co-pigmentation produit par ajout d'une flavone et de malvidine par une technique de modification génétique par expression d'un gène de l'anthocyanine méthyltransférase, d'un gène de la flavone synthase et du gène de la flavonoïde 3',5'-hydroxylase de pensée *(Viola x wittrockiana).*

15. Le procédé selon la revendication 14, dans lequel l'effet de co-pigmentation est un effet de changement de la couleur de la fleur vers le bleu.
